(19) Europäisches Patentamt
European Patent Office
Office européen des brevets

(11) Publication number : **0 330 615 B1**

(12) # EUROPEAN PATENT SPECIFICATION

(45) Date of publication of patent specification : **15.03.95 Bulletin 95/11**

(51) Int. Cl.$^6$ : **G02B 1/04,** C08G 65/28, C08G 65/32

(21) Application number : **89810123.3**

(22) Date of filing : **16.02.89**

(54) **Wettable, flexible, oxygen permeable, contact lens containing polyoxyalkylene backbone units, and use thereof.**

(30) Priority : **26.02.88 US 160622**

(43) Date of publication of application : **30.08.89 Bulletin 89/35**

(45) Publication of the grant of the patent : **15.03.95 Bulletin 95/11**

(84) Designated Contracting States : **AT BE CH DE ES FR GB GR IT LI LU NL SE**

(56) References cited :
**EP-A- 0 109 355**
**EP-A- 0 263 061**

(73) Proprietor : **CIBA-GEIGY AG**
**Klybeckstrasse 141**
**CH-4002 Basel (CH)**

(72) Inventor : **Su, Kai Chiang**
**13090 Hopewell Road**
**Alpharetta Georgia 30201 (US)**
Inventor : **Molock, Frank**
**1535 Summer Hollow Trail**
**Lawrenceville Georgia 30245 (US)**

EP 0 330 615 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to ophthalmic devices, such as contact lenses and intraocular implants, and particularly contact lenses of a polymer containing a backbone containing polyoxyalkylene units possessing a unique blend of properties including a) high oxygen permeability, b) good wettability, c) flexibility and d) optical clarity, in the ocular environment of use.

The use of optical contact lenses for the correction of vision defects or for cosmetic purposes is well known. However, existing contact lenses have been found to be unacceptable to many potential contact lens patients for a variety of reasons. For example, early contact lenses were made from polymethyl methacrylate (PMMA). While PMMA lenses have high optical clarity and good durability, they are rigid lenses possessing low oxygen permeability. Consequently, PMMA lenses may result in eye irritation and corneal oxygen deprivation leading to wearer intolerance and limiting the usefulness of such lenses.

In an attempt to avoid these problems, so-called "soft" lenses, capable of swelling in an aqueous environment, were developed. These "soft" or hydrogel lenses, characteristically made from poly (2-hydroxyethyl methacrylate), poly (vinyl alcohol) or poly (vinylpyrrolidone) generally result in less irritation and intolerance than PMMA lenses for most patients. When substantial amounts of water are absorbed into the hydrogel, the oxygen permeability is increased over that of PMMA lenses, and the flexibility of such hydrogel lenses is high, thereby increasing patient comfort. However, the oxygen permeability of such hydrogel lenses is generally still rather low, and the durability is poor. Moreover, due to the high water content of such lenses, they generally have a tendency to collect and trap proteinaceous and other tear fluid materials, resulting in lens clouding over a period of time.

In another attempt to solve problems associated with early lenses, silicone, or siloxane, rubber lenses were developed. They are advantageous in that they possess high oxygen permeability and an aesthetically appealing texture when worn. However, due evidently to the generally low thermal conductivity of silicone rubber, burning sensations in wearers of silicone rubber lenses have been reported. Also, as silicone lenses tend to be lipophilic, such lenses may tighten onto the cornea, trapping debris between the lens and cornea, thereby resulting in corneal abrasions. Also, due to the characteristic lipophilic nature of such lenses, the silicone rubber is mucophilic and non-wettable, attracting ocular debris such as proteins, lipids, mucoids and the like.

It is an object of the present invention to overcome these and other disadvantages of the art by providing substantially siloxane free, wettable, oxygen permeable, ophthalmically acceptable polymer and ophthalmic devices, such as contact lenses and corneal implants, of a polymer containing polyoxyalkylene backbone units.

A further object of the invention is to provide a method of correcting visual defects in the form of refractive errors by fitting to the patient's eye in need of the same a corrective contact lens of such polymer.

These and other objects of the invention are apparent from the following detailed description of the invention.

One embodiment of the present invention relates to an optically clear, hydrolytically stable, biologically inert, wettable, flexible, substantially siloxane free, swellable in aqueous ocular tear fluid, oxygen permeable ophthalmic device, such as a contact lens, fabricated from a crosslinkable polymer containing segments of the formula I

$$[-A-L-D-]_w \qquad (I)$$

wherein each w is 1-8;

each D is -O- or $-NR^7-$ in which $R^7$ is hydrogen, lower alkyl, or phenyl;

each L is independently selected from -B-R-B′-, -BR- or RB′-, wherein each B and B′ is independently selected from

$$-\overset{O}{\overset{\|}{C}}-, \quad -\overset{O}{\overset{\|}{C}}-O-, \quad \text{and} \quad -\overset{O}{\overset{\|}{C}}NH$$

with the carbonyl group thereof being bound to A or D;

each R is a divalent linking group selected from

a) a divalent aliphatic group, preferably alkyl, alkenyl, or alkynyl, of up to 25 carbon atoms which may be interrupted by an interrupting unit selected from oxy, carbonyloxy, amino, aminocarbonyl, oxycarbonyl, ureido, oxycarbonylamino, and carbonylamino;

b) a divalent $-(\text{aliphatic})_{alpha}$-5-7 membered alpha cycloaliphatic-$(\text{aliphatic})_{beta}$-group wherein each of alpha and beta is independently 0 or 1, and each of the non cyclic aliphatic groups is independently selected from group a) above, said group b) having up to 25 carbon atoms;

c) a divalent $-(\text{aliphatic})_{alpha}$-aryl-$(\text{aliphatic})_{beta}$-group having 6 to 25, preferably 7-16 carbon atoms in the aryl portions; and each of the aliphatic groups are independently selected from group a) above and alpha

and beta are independently 0 or 1, wherein groups b) and c) can be interrupted in the non-cyclic portions thereof or between the cyclic and non-cyclic portions thereof by an interrupting group as defined in a) above, and groups c) may be further substituted with one or more substituents selected from halogen, preferably fluorine or chlorine, $C_{1-4}$ alkyl, preferably methyl, and $C_{1-12}$ perhaloalkyl, especially $C_{1-12}$ perfluoralkyl;

and each A is a copolymeric block of polyoxyalkylene, of which at least 30 %, preferably at least 50 %, more preferably at least 75 %, most preferably 100 % of the A groups are

aa) homopolymer blocks of the formula

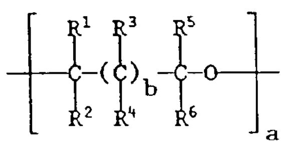

(A)

wherein b is 0 to 4, a is up to 15, preferably not greater than 10, more preferably not greater than 7, most preferably not greater than 4, and $R^1$-$R^6$ are as defined hereinafter and

ab) copolymer blocks of the formula

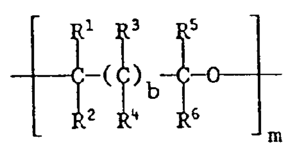

(B)

wherein b is 0 to 4, $R^1$-$R^6$ are as defined hereinafter,

and m is up to 1000 provided that within m, there is no homopolymeric block of more than 15 oxyalkylene units, preferably of no more than 10 units, more preferably of no more than 7 units, most preferably of no more than 4 units per sub block, the remaining groups being homopolymers or copolymers of the formula

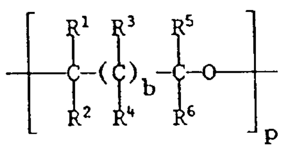

(C)

wherein b is 0 to 4, p is up to 1000 without regard for the size of the homopolymeric sub-blocks within p; the terminal oxygen within the A group being eventually replaced by $-NR^7-$ and wherein each of $R^1$, $R^2$, $R^5$ and $R^6$ is independently selected from the group consisting of hydrogen, an aliphatic, aromatic or heterocyclic radical selected from unsubstituted $C_1$-$C_{16}$ alkyl; substituted $C_1$-$C_{16}$ alkyl; unsubstituted $C_2$-$C_{16}$ alkenyl; and substituted $C_2$-$C_{16}$ alkenyl; wherein the alkyl and alkenyl substituents are independently selected from $C_1$-$C_{16}$ alkoxycarbonyl, $C_2$-$C_{16}$ alkenyloxycarbonyl, fluoro, aryl of up to 10 carbon atoms, $C_1$-$C_8$ alkoxy, $C_2$-$C_6$ alkanoyloxy, aryloxy of up to 10 carbon atoms, $C_3$-$C_6$ alkenoyloxy, aroyloxy of up to 11 carbon atoms, $C_3$-$C_8$ cycloalkyl, $C_3$-$C_8$ cycloalkoxy, $C_3$-$C_8$ cycloalkyl-carbonyloxy, $C_3$-$C_8$ cycloalkoxy-carbonyl, oxacycloalkyl of up to 7 carbon atoms, oxacycloalkoxy of up to 7 carbon atoms, oxacycloalkoxy (up to 7 carbon atoms)-carbonyl, oxacycloalkyl (up to 7 carbon atoms)-carbonyloxy, and aryl (of up to 10 carbon atoms)-oxycarbonyl, each of said alkyl and alkenyl substituents being, in turn, optionally substituted by $C_1$-$C_6$ alkyl, fluoro or a $C_1$-$C_6$ alkoxy provided said last mentioned alkoxy is not bound to a carbon atom already bound to another oxygen atom; $R^1$, $R^2$ , $R^5$ and $R^6$ being further independently selected from aryl of up to 10 carbon atoms, $C_3$-$C_8$ cycloalkyl, and oxacycloalkyl of up to 7 carbon atoms, each of which may be unsubstituted or further substituted with a substituent selected from the group of substituents for said alkyl set forth above;

each $R^3$ and $R^4$ are selected from the same group set forth above for $R^1$; and $R^3$ and $R^4$ are further independently selected from $C_1$-$C_{16}$ alkoxycarbonyl, $C_2$-$C_{16}$ alkanoyloxy, $C_2$-$C_{16}$ alkenyloxycarbonyl, and $C_3$-$C_{16}$ alkenoyloxy, each of which may be further substituted by fluoro, aryl of up to 10 carbon atoms, or $C_1$-$C_{16}$ alkoxy, and $R^3$ and $R^4$ are still further independently selected from aryloxy of up to 10 carbon atoms, cycloalkoxy of up to 8 carbon atoms, cycloalkyl (of up to 8 carbon atoms)-carbonyloxy, cycloalkoxy (of up to 8 carbon atoms)-carbonyl, aroyloxy of up to 11 carbon atoms, oxacycloalkoxy of up to 7 carbon atoms, oxacycloalkenyloxy of up to 7 carbon atoms, oxacycloalkoxy (of up to 7 carbon atoms)-carbonyl, oxacycloalkyl (of up to 7 carbon

atoms)-carbonyloxy, oxacycloalkenyloxy (of up to 7 carbon atoms)-carbonyl, and aryloxy (of up to 10 carbon atoms)-carbonyl, each of which may be further substituted by fluoro, $C_1$-$C_6$ alkyl or $C_1$-$C_6$ alkoxy, provided that any substituent having an oxygen atom or carbonyl group thereof as its link to the rest of the molecule may not be a substituent on the same carbon atom which is bonded to another oxygen atom, in addition, 2 adjacent groups selected from $R^1$ to $R^6$, together with the atoms to which they are attached may form a 5 - 8 membered cycloalkyl, 5 - 8 membered oxacycloalkyl or bicycloalkyl ring, such that the resulting polymer absorbs more than 10 % by weight water based upon the total weight of polymer.

When each b is zero, at least one of $R^1$, $R^2$, $R^5$ and $R^6$ in at least a portion of the segments having formula I is other than hydrogen. The polymer is sufficiently hydrophilic that it exhibits a receding contact angle with distilled water at 20°C of less than 60°, preferably less than 40°, more preferably less than 25°, even more preferably less than 15°, most preferably less than 10°.

In the foregoing, all alkyl groups whether mentioned alone or as part of another group are preferably $C_1$-$C_4$ alkyl, such as methyl, ethyl, propyl and butyl, especially t-butyl, with the exception that adjacent groups on aryl rings cannot each be t-butyl. These alkyl groups may be straight chain or branched chain. When the alkyl is a substituent on a phenyl ring, it is preferably attached at the para position. Preferably alkenyl groups, whether alone or as part of another group are $C_2$-$C_4$ alkenyl, such as ethenyl, propenyl and butenyl. Preferred aryl groups (whether alone or as part of another group) are phenyl and naphthyl, more preferably phenyl. Preferably the aryl groups are still further substituted by $C_1$-$C_4$ alkyl, more preferably t-butyl, most preferably in the para position.

A preferred embodiment is a polymer as hereinbefore disclosed wherein said -A- groups are selected from formula A and formula C, or from formula B and formula C.

Each A group can have from 1 to 1000 oxyalkylene units, preferably 1-200, more preferably 1-150 units, still more preferably 1-100 units, yet more preferably 1-75 units, most preferably 1-50 units.

Two most preferably embodiments are when at least 30 % of the A groups in the polymer consists of 32 % and 35 % polyethylene glycol and 68 % and 65 %, respectively of polypropylene glycol, the polyethylene glycol blocks being of no more than three repeating units each and the polypropylene glycol blocks being of no more than seven repeating units each.

Preferably each b is independently 0 - 3, most preferably 1 to 2. A further preferred embodiment is that the group identified by formula I be at least 25 % halogen free, preferably 30 %, still more preferably 40 %, even more preferably substantially halogen free and most preferably, totally halogen free. Wherever cyclo groups are indicated, whether carbocyclic or heterocyclic they preferably have 5 - 6 ring members and the heterocyclics preferably have only carbon atoms and one oxygen atom as ring members.

In formula A, B or C, when b is greater than one, each of the multiple $R^3$ and $R^4$ groups may be the same or different; however preferably all of the $R^3$ groups are the same and all of the $R^4$ groups are the same.

Preferably, each b is independently an integer of 0 to 2, and most preferably zero or one.

In one particularly preferred aspect of the invention, each of $R^1$-$R^5$ is hydrogen, and if each b within an A group is zero, then at least a portion of the $R^6$ groups are methyl.

Preferably the substituents for $R^6$ are alkyl of up to 16 carbon atoms; alkyl of up to 16 carbon atoms substituted by alkoxy of up to 8 carbon atoms, or fluoro; phenyl which is unsubstituted or substituted by fluoro, alkoxy of up to 6 carbon atoms or alkyl of up to 6 carbon atoms; benzyl wherein the phenyl ring thereof is unsubstituted or substituted by fluoro, alkoxy of up to 6 carbon atoms or alkyl of up to 6 carbon atoms; cyclohexyl; or oxacycloalkyl of 4 or 5 ring carbon atoms.

A highly advantageous subembodiment relates to wettable ophthalmic devices, preferably contact lenses, fabricated from a polymer containing as A-groups of formula A, B or C segments of the formula II

$$-\left[ [CH_2-(CH_2)_d-\overset{\overset{R^6}{|}}{C}H-O]_f-[CH_2-(CH_2)_{d'}-\overset{\overset{R^6}{|}}{C}H-O]_{f'} \right]_q- \qquad II$$

wherein each d and d' is selected from the same group as b in formula A, B or C and each f and f' cannot exceed 15 and (f+f') times q equals a, m or p, respectively.

Preferably, at least one $R^6$ within each formula II is an aliphatic, aromatic, or heterocyclic radical, preferably alkyl of up to 6 carbon atoms, alkyl of up to 6 carbon atoms substituted by alkoxy of up to 6 carbon atoms or fluoro; phenyl which is unsubstituted or substituted by fluoro, alkoxy of up to 6 carbon atoms or alkyl of up to 6 carbon atoms; benzyl wherein the phenyl ring thereof is unsubstituted or substituted by fluoro, alkoxy of up to 6 carbon atoms or alkyl of up to 6 carbon atoms; cyclohexyl or oxacycloalkyl of 4 or 5 ring carbon atoms.

Free hydroxy groups on the outer surfaces of the formed polymer are more preferable than in the interior of the polymer in that they increase wettability without drawing water into the polymer matrix. A suitable means of tying up the free hydroxy groups present would be to interact them with a color group. Typical color groups useful in these embodiments include, but are not limited to the hydroxy-reactive dyes known in the art under the tradename Remazol, manufactured by American Hoechst.

Examples of the Remazol dyes which are especially suitable are:

| Dye | Color Index Code |
|---|---|
| Remazol Brill Blue RW | Reactive Blue 19 |
| Remazol Yellow GR | Reactive Yellow 15 |
| Remazol Black B | Reactive Black 5 |
| Remazol Golden Orange 3GA | Reactive Orange 78 |
| Remazol Turquoise P | Reactive Blue 21 |

all of which have at least one group of the formula

$$-SO_2-CH_2CH_2O-SO_3^{\ominus}$$

which reacts with the polymer or monomer hydroxy group to yield a dye-$SO_2$-$CH_2$-$CH_2$-O-polymer or

$$\text{dye-}SO_2\text{-}\overset{CH_3}{\overset{|}{C}}H\text{-O-polymer}$$

group, preferably the former. In such a manner, both excess free hydroxy groups are disposed of and colored contact lenses can be realized simultaneously. Another means of disposing of these excessive hydroxy groups is to utilize their presence to form various degrees and types of crosslinking.

In a further embodiment of the invention, the ophthalmic device, preferably a contact lens, is fabricated from a polymer characterized in that the units of the formula I are part of a monomer of the formula

$$L'\text{-}D\text{-}[A\text{-}L\text{-}D]_w\text{-}A\text{-}L'' \qquad \text{(III)}$$

wherein A and w are as defined hereinbefore, provided that not all A groups in any one polymer can be homopolymers of polyethylene glycol or polypropylene glycol;
wherein each D is independently oxygen or -N($R^7$)-; each $R^7$ is independently selected from hydrogen, $C_1$-$C_4$ alkyl and phenyl;
wherein each L is independently selected from -BRB′-; wherein B, R and B′ are as defined hereinbefore;
wherein L′ is selected from hydrogen, P′-B-$R^x$-B′-, and P′-$R^x$-B′-, wherein each $R^x$ is independently selected from

a) a divalent aliphatic group, preferably alkyl, alkenyl, or alkynyl, of up to 25 carbon atoms which may be interrupted by an interrrupting unit selected from oxy, carbonyloxy, amino, aminocarbonyl, oxycarbonyl, ureido, oxycarbonylamino, and carbonylamino;
b) a divalent 5-7 membered cycloaliphatic group having 5 to 25 carbon atoms;
c) a divalent arylene group having 6 to 25, preferably 7-16 carbon atoms;
d) a divalent aralkyl or alkaryl group having 7 to 25, preferably 8 to 16 carbon atoms;

wherein groups b) and d) can be interrupted in the non-cyclic portions thereof or between the cyclic and non-cyclic portions thereof by an interrupting group as defined in a) above, and groups c) and d) may be further substituted in the aryl portions thereof with one or more substituents selected from halogen, preferably fluorine or chlorine, $C_{1-4}$ alkyl, preferably methyl, and $C_{1-12}$ perhaloalkyl, especially $C_{1-12}$ perfluoralkyl;
with the carbonyl group of B being bound to P′, and P′ is hydrogen, amino, hydroxy, or a moiety containing a crosslinkable group which may be crosslinked when coreacted with a suitable crosslinking agent or when irradiated by actinic radiation;
and L″ is selected from hydrogen, L‴ as defined hereinafter, -B-$R^x$-B′-P′, and -B-$R^x$-P′, wherein $R^x$ and P′ are as defined above except that the carbonyl of B′ instead of B is bound to P′; and L‴ is a terminal monovalent aliphatic, aromatic or cycloaliphatic group of up to 14 carbon atoms.

Preferably P′ is a vinyl containing moiety.

When P′ is a vinyl containing group for example

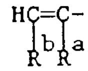

with one of $R^a$ and $R^b$ being methyl or hydrogen and the other being hydrogen, then the monomer of formula III can be crosslinked in the presence or absence of up to about 10 % of other vinylic comonomers, provided that such comonomers are substantially free of hydroxy groups in the final product.

The vinylic comonomer is frequently utilized to increase the hydrophilicity of the final product without substantially altering the other properties mentioned above. Typically, when the vinylic comonomer is a polyethylene glycol of the formula

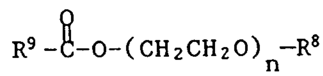

or pyrrolidone of the formula

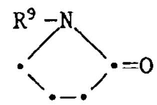

with n being 1-25, $R^8$ being hydrogen or methyl and $R^9$ being $CH_2=CH-$, $CH_2=C(CH_3)-$ or other UV curable moiety, the resultant polymer is more hydrophilic than previously, but the Dk is essentially the same as when the comonomer is absent.

Usually, when present, the vinylic comonomer is used in an amount of about 2 % to about 10 % by weight of the resultant polymer. Advantageously, no more than 5 % of vinylic comonomer is used when the compound of formula III has a molecular weight in excess of 8000. Generally, when the compound of formula III has a molecular weight of under about 4000, up to 10 % by weight of vinylic comonomer can be used. When the compound of formula III has a molecular weight between 4,000 and 8,000, the maximum amount of vinylic comonomer is between 5 % and 10 % by weight.

When P′ does not have a vinylic group, but takes part in crosslinking, P′ contains e.g. an active hydrogen. In such cases, P′ preferably terminates in an OH, $NHR^c$ ($R^c$ being hydrogen or lower alkyl), a leaving group bound directly to the B or B′ carbonyl, a conventional acyl leaving group when not so bound, SCN- or OCN-. Crosslinking is then typically carried out by condensation or addition with a di- or polyfunctional coreactive monomer. For example, when P′ is OH, then the coreactive monomer functional group can be $-NHR^c$, -COOH, OCN-, SCN-, etc.; when P′ is $NHR^c$, the reactive comonomer functional group can be a conventional acyl, or acyl bound to a conventional leaving group; and when P′ has OCN- or SCN-, then the reactive comonomer functional group can be OH. Similarly, the other coreactive functional groups mentioned in terms of either P′ or the coreactive monomer can be interchanged (those mentioned as part of P′ being on the coreactive monomer and those mentioned as part of the coreactive monomer being part of P′).

Suitable vinylic comonomers and coreactive monomers for condensation are set forth below. However, the list is not exhaustive and those of ordinary skill will appreciate the modifications, additions, and alternatives which may also be employed.

Minor amounts i.e. less than 50 %, preferably up to 30 %, and most preferably up to no more than about 10 % by weight, of conventional copolymerizable vinyl monomers can be employed as extenders or hydrophilic modifiers, or the like, in the preparation of the instant polymer, as copolymer constituents. Suitable vinyl monomers include: acrylates and methacrylates of the general formula

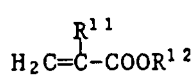

where $R^{11}$ is hydrogen or methyl and $R^{12}$ is a straight chain or branched aliphatic, cycloaliphatic or aromatic group having up to 20 carbon atoms which is unsubstituted or substituted by one or more alkoxy, alkanoyloxy or alkyl of up to 12 carbon atoms, or by halo, especially chloro or preferably fluoro, or $C_3$-$C_5$ polyalkyleneoxy of 2 to about 100 units; acrylamides and methacrylamides of the general formula

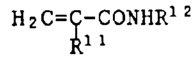

where $R^{11}$ and $R^{12}$ are as defined above;
vinyl ethers of the formula

$$H_2C=CH\text{-}O\text{-}R^{12}$$

where $R^{12}$ is as defined above;
vinyl esters of the formula

$$H_2C=CH\text{-}OOC\text{-}R^{12}$$

where $R^{12}$ is as defined above;
maleates and fumarates of the formula

$$R^{12}OOC\text{-}HC=CH\text{-}COOR^{12}$$

where $R^{12}$ is as defined above;
and vinylic substituted hydrocarbons of the formula

$$R^{11}CH=CHR^{12}$$

where $R^{11}$ and $R^{12}$ are as defined above.

Useful monomers include, for example:
methyl-, ethyl-, propyl-, isopropyl-, butyl-, ethoxyethyl-, methoxyethyl-, ethoxypropyl-, phenyl-, benzyl-, cyclohexyl-, hexafluoroisopropyl-, or n-octyl-acrylates and -methacrylates as well as the corresponding acrylamides and methacrylamides;
dimethylfumarate, dimethylmaleate, diethylfumarate, methyl vinyl ether, ethoxyethyl vinyl ether, vinyl acetate, vinyl propionate, vinyl benzoate, acrylonitrile, styrene, alphamethyl styrene, 1-hexene, vinyl chloride, vinyl methyl ketone, vinyl stearate, 2-hexene and 2-ethylhexyl methacrylate.

As hydrophilic modifiers, to increase hydrophilicity without substantial loss of Dk, the vinyl comonomer can be a N-(vinyl containing group)-pyrrolidone or a polyoxyethylene (of 1-25 repeating units) acrylate or methacrylate, or other hydrophilic modifier as defined by the copending EP-A-331 633 entitled "HYDROPHILIC MODIFIER MONOMERS" by inventors Frank Molock, Richard Robertson, and Kai Su.

When either or both L′ and L″ are hydrogen, or terminate in P′ with P′ being hydrogen, at least one additional crosslinkable moiety must be present as one of, or as substituent on one of, the groups $R^1$-$R^6$. Such crosslinkable groups may also be present as a substituent on or in place of one or more of $R^1$-$R^6$ even when both of L′ and L″ have crosslinkable groups therein. However, the degree of crosslinking in the finished crosslinked polymer should not exceed 10 %, preferably not more than 5 %, more preferably be in the range of 1-4 %, most preferably in the range of 2-3 %.

In a preferred embodiment, A is the divalent moiety according to formula II.

Especially preferred are polymers of the monomer according to formula III in which L′ and/or L″ is of formula IV

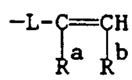
(IV)

wherein $R^a$ and $R^b$ are each independently hydrogen or methyl, but not simultaneously methyl; and L is -BRB′-, -BR- or -RB′-.

A valuable sub-embodiment of the invention relates to ophthalmic devices, preferably contact lenses, of polymers consisting essentially of polymerized units of the formula V

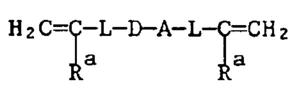
(V)

wherein $R^a$ and A are as defined above, each $R^a$ being independent of the other and L is -BRB′-, -BR- or -RB′-. Particularly preferred are polymers of the monomers of formula IV and V wherein L is -BRB′-, R is a divalent arylene group of 6 to 14 carbon atoms, or is a divalent $C_2$-$C_6$ alkylene-oxycarbonylamino-$C_6$-$C_{10}$-arylene group; D is oxygen, and B and B′ are each -NHCO- wherein the nitrogens thereof are directly bonded to R. Even particularly preferred are polymers of the monomers of formula IV and V wherein L is -BR- or -RB′-, respectively,

R is a divalent arylene of 6 to 14 carbon atoms, D is oxygen, and B and B′ are each -NHCO- wherein the nitrogens thereof are directly bonded to R.

Also highly preferred are those polymers of monomers of formula III and V wherein A is of the formula VI

$$\left[-CH_2\overset{R^{10}}{\overset{|}{C}}HO-\right]_x\left(\left[-CH_2CH_2O-\right]_{y'}\left[-CH_2\overset{R^{10}}{\overset{|}{C}}HO-\right]_{x'}\right)_z\left[-CH_2CH_2O-\right]_y \qquad (VI)$$

wherein one of x and y is zero and the other and y′ and x′ are independently 1-20, z is a number such that x+y+zy′+zx′ = 4 to 1000 and $R^{10}$ is alkyl of 1 to 4 carbon atoms. Another valuable embodiment requires the value of x′ to be at least about twice that of y′ and $R^{10}$ to be alkyl of 1 to 4 carbon atoms, preferably methyl.

The above reactive vinylic monomers are characteristically polymerized under conventional polymerization conditions. In those vinylic monomers containing but one vinyl group, a minor amount e.g. from about 0.01 to about 5 weight percent, based on the monomer of formula III, of a conventional crosslinking agent, may be employed. Suitable crosslinking agents include diolefinic monomers such as:

Allyl acrylate and methacrylate, alkylene glycol and polyalkylene glycol di-acrylates and -methacrylates, such as ethyleneglycol dimethacrylate, diethylene glycol dimethacrylate, and propylene glycol dimethacrylate; trimethylol propane triacrylate; pentaerythritol tetraacrylate, divinyl-benzene; divinyl ether; divinyl sulfone; bisphenol A diacrylate or -methacrylate; methylene bisacrylamide; diallyl phthalate; triallyl melamine and hexamethylene di-acrylate and -methacrylate. Also, such minor amounts of a crosslinking agent may be employed, if desired, in the polymerization of the di-vinyl monomer of formula III and V.

When the monomers of formula III have free hydroxy, isocyanato, carboxylic or amine groups, suitable crosslinking agents contain di- or polyfunctional co-reactive groups to form addition or condensation reactions linking 2 or more chains.

If desired, the monomer reaction mixture may contain a catalytic amount of a conventional catalyst, preferably a free radical catalyst. Of particular interest are conventional peroxide and azo catalysts, such as hydrogen peroxide, benzoyl peroxide, tert-butyl peroctoate, benzoyl peroxide or azobis(isobutyronitrile).

The polymerization can generally be carried out at temperatures between about 20° and about 150°C, for a period between about 1 and about 24 hours. It is understood that the time and temperature in such a reaction are inversely related. Thus, temperatures employed in the upper end of the temperature range will generally provide reaction times near the lower end of the time range. Preferably, the polymerization is conducted in the presence of actinic radiation, such as UV light.

Depending upon the nature of the polymer mixture, it may be desirable for the polymers obtained from such polymerizations to be post cured, e.g. at a somewhat elevated temperature such as between about 60°C and about 150°C.

For the preparation of contact lenses, the polymer mixture may be cast directly in the shape of the lens, or the polymerization may be carried out in a mold having a shape convenient for further processing, such as in the shape of small cyclinders or "buttons", which can then be machined.

In yet a further subembodiment of the invention, the ophthalmic device, preferably a contact lens, is fabricated from a polymer consisting essentially of an addition product of formula VII

$$E-Y^1-D-(A-L-D)_w-A-Y^1-E \qquad (VII)$$

and a compound of formula VIII,

$$(E^1)_t\text{-}G \qquad (VIII)$$

wherein A, L, D and w are as defined above;
t is an integer of 2 to 4;
G is an aliphatic, aromatic, araliphatic, carbocyclic or heterocyclic residue having a valency corresponding to the value of t and containing up to about 24 carbon atoms, or, where t is 2, may also represent a divalent group of the formula

$$-Y^1-D-(A-L-D)_w-A-Y^1- \quad ;$$

$Y^1$ is a divalent aliphatic group of up to 14 carbon atoms which may be interrupted by oxy, carbonyloxy, amino,

aminocarbonyl, oxycarbonyl, ureido, oxycarbonylamino or carbonyl; a divalent aliphatic hydrocarbyl-carbonyl or -aminocarbonyl group of up to 14 carbon atoms and wherein the carbonyl group thereof is covalently bonded to the adjacent oxygen or $-N(R^7)-$ moiety; a divalent 5 to 7-membered cycloaliphatic group of from 5 to 14 carbon atoms; a divalent arylene group of 6 to 14 carbon atoms; a divalent aralkyl or alkaryl group of 7 to 14 carbon atoms; a divalent 5 to 7-membered cycloaliphatic-carbonyl or -aminocarbonyl group of from 6 to 15 carbon atoms, wherein the carbonyl group thereof is covalently bonded to the adjacent oxygen or $-N(R^7)-$ moiety; or a divalent arylene-, aralkyl- or alkaryl-carbonyl or -aminocarbonyl group wherein the arylene group is of 6 to 14 carbon atoms, the aralkyl or alkaryl group is of 7 to 14 carbon atoms, and the carbonyl group is covalently bonded to the adjacent oxygen or $-N(R^7)-$ moiety; or $Y^1$ is a direct bond where E is hydrogen;
E is hydrogen, hydroxy or amino when $E^1$ is isocyanato or isothiocyanato; and E is isocyanato or isothiocyanato when $E^1$ is hydroxy or amino.

Advantageously, in order to insure adequate crosslinking, in one sub-embodiment there is employed at least a minor amount of those compounds wherein t is 3, for example at about 0.2 % by weight based upon the amount of compound of formula IV employed. Generally, a stoichiometrically equivalent amount of the compounds of formula VII and VIII is combined; however a slight excess of di- or polyisocyanate or -isothiocyanate may be employed to insure sufficient crosslinking to maintain dimensional stability in the product. As a further alternative, additional conventional crosslinking agents may be employed to insure sufficient crosslinking such that the product maintains dimensional stability. Thus, in addition to the compounds of formula VII and VIII there may also be added to the reaction mixture a minor amount, e.g. up to about 5 weight percent, of a conventional di-isocyanate or tri-isocyanate such as toluene di-isocyanate, isophorone di-isocyanate, 4,4′-methylenebis(phenyl isocyanate), methylenebis(cyclohexyl isocyanate), melamine tri-isocyanate, and the like. Alternatively, where a stoichiometric excess of isocyanate is employed in the reaction of VII and VIII, a minor amount, e.g. up to about 5 weight percent, of a di- or polyfunctional amine or hydroxylated crosslinking agent may be employed. Suitably such crosslinking agents include, for example ethylene glycol, glycerin, diethylene glycol, ethylene diamine, ethanolamine, triethanolamine, diethanolamine and the like.

The addition reaction between the compounds of formula VII and formula VIII and any additional crosslinker can be conducted under conditions known, per se. Thus, the compounds may be simply admixed, in the presence of an inert diluent if necessary or desired, at a reaction temperature between about 0°C and about 100°C, preferably between about 20°C and 80°C, optionally in the presence of a condensation catalyst, such as triethyl amine or di-n-butyltin diacetate.

In the preparation of ophthalmic devices, such as contact lenses, the reaction mixture may be cast directly in the shape of the lens, or the polymerization may be carried out in a mold having a shape convenient for further processing, such as the shape of a small cylinder or "button", which can then be machined.

The compounds of the formula III, V, VII and VIII are either known or can be prepared by methods which are known, per se.

For example, the vinylic monomers of formula V can be prepared by reacting either

(a) a mono-ol of the formula IX

$$HO-(A-L-D)_w-A-L''' \qquad (IX)$$

where L‴ is a terminal monovalent aliphatic, aromatic or cycloaliphatic group of up to 14 carbon atoms, or

(b) a diol of the formula X

$$HO-(A-L-D)_w-A-H \qquad (X)$$

wherein A, L, D and w are as defined above, with a stoichiometric amount of a vinylic compound of the formula XI

$$H_2C=\underset{R^a}{\underset{|}{C}}-B-R-X \qquad (XI)$$

wherein X is an isocyanato group; an activated carboxy group, such as an anhydride, an acid halide, or a carboxy ester; or is a leaving group, such as a halide, sulfato, or the like; at temperatures between about 0°C and about 100°C, in the presence or absence of a conventional additional catalyst, and in the optional presence of an inert diluent, and recovering the product of formula V. Where X is a leaving group, such as a halide, the product of formula X may be in the form of its alkoxide, such as alkali metal alkoxide, salt.

Alternatively, one may prepare products of formula III wherein B and B′ are -NHCO- and D is -O- by reacting

a diisocyanate, such as an aliphatic, aromatic, cycloaliphatic, or araliphatic diisocyanate with a mono-ol or diol according to formula IX or X, respectively, and react the corresponding isocyanato terminated product with a hydroxy containing acrylate or methacrylate, such as hydroxyethyl-acrylate or -methacrylate, or an allyl amine or methallyl amine or allyl or methallyl alcohol to form the corresponding product of formula III at a temperature between about 0°C and 100°C, in the presence or absence of an inert diluent, and optionally in the presence of an addition catalyst, such as a tertiary amine, e.g. triethylamine or an organo-tin compound and recovering the product of formula III.

Still further, compounds of formula X can be reacted with compounds of the formula XII

$$X\text{-}R\text{-}X \qquad (XII)$$

where R and X are as defined above, to form a compound of the formula XIII

$$X\text{-}R\text{-}B\text{-}D\text{-}(A\text{-}L\text{-}D)_w\text{-}A\text{-}B\text{-}R\text{-}X \qquad (XIII)\ .$$

Compounds of formula XIII are then reacted with an X coreactive moiety which also contains a vinyl group, for example hydroxy ethyl methacrylate to yield a compound of formula III.

The compounds of formula IX and formula X are known or can easily be prepared by methods known per se.

For example, the compounds of formula X can be prepared by reacting a diol of the formula HO-A-H with a difunctional reactive group containing compound having the group L wherein the reactive groups are isocyanate, activated carboxy, such as an anhydride, an acid halide or a carboxy ester, or a leaving group, such as halide, sulfato or the like. Where the molar ratio of diol to the difunctional reactive group containing compound is about 2 to 1, the value of w in the resulting adduct of formula X is about 1; where about 3 moles of diol are reacted with about 2 moles of the difunctional group containing compound, the resulting average value of w in the adduct of formula X is characteristically about 2, and so on. The aforementioned reaction to obtain those compounds of formula X where w is 1 or greater, can be conducted at a reaction temperature between about -10°C and about 100°C depending on the relative reactivities of the species involved, in the presence or absence of an inert diluent and in the optional presence of an addition catalyst, if desired or appropriate.

Suitable polyols and amino polyethers of the formula HD-A-H wherein A represents the divalent moiety of formula Ia are generally known materials or can be prepared by methods known, per se.

Thus, the polyols of the formula HO-A-H are generally prepared by the addition reaction of an epoxide of the formula XIV

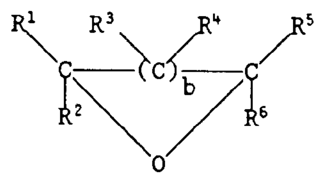

(XIV)

where $R^1$-$R^6$ and b are as defined above, with another epoxide of formula XIV, optionally in the presence of a conventional alkylation catalyst, at atmospheric to elevated pressures of up to about 3000 kPa gauge, at temperatures between 0°C and about 130°C, optionally in the presence of an inert diluent. If desired, one may add to the reaction mixture, prior to the reaction of the epoxides, an aliphatic, aromatic or cycloaliphatic alcohol, acid or amine having up to 14 carbon atoms to prepare the corresponding mono-ols terminating in the group D.

The reaction between the epoxides, when mixtures of different epoxides are employed to obtain the polyol of the formula HO-A-H, can be conducted by admixing the epoxides to obtain random copolymers or terpolymers, etc., or the addition can be conducted sequentially to form block copolymers having terminal hydroxy groups. Suitable catalysts include alkaline earth oxides, alkaline earth carbonates, alkyl zinc compounds, aluminum alkoxides, hydrates of ferric chloride, bromide and acetate, and gamma radiation. The reaction may also be initiated by the presence of a glycol, such as ethylene glycol or propylene glycol or by a polyol of higher functionality such as sucrose, or by an amine, such as ethylene diamine, toluenediamine, and so forth. Generally the length of time of the reaction will depend in part on the alkylene oxide employed, but can generally be from less than one to several score hours. Thus, ethylene oxide generally is about three times as active as propylene oxide, which in turn reacts more rapidly than 1,2-butylene oxide. The preparation of polyoxetanes and polytetrahydrofurans is generally initiated via ring opening oxonium formation using trialkyloxonium salts, carboxonium salts, acylium salts and the like.

Suitable diols of the formula HO-A-H include those prepared from epoxides such as: 1,2-propylene oxide; 1,2-butylene oxide; 1,2-epoxydecane; 1,2-epoxydodecane; 1,2-epoxyoctane; 2,3-epoxynorbornane; 1,2-epoxy-3-ethoxypropane; 1,2-epoxy-3-phenoxypropane; 2,3-epoxypropyl 4-methoxyphenyl ether; tetrahydrofuran; 1,2-epoxy-3-cyclohexyloxypropane; oxetane; 1,2-epoxy-5-hexene; 1,2-epoxyethylbenzene; 1,2-epoxy-1-methoxy-2-methylpropane; perfluorohexylethoxypropylene oxide; benzyloxypropylene oxide, and the like. Also, the aforementioned epoxides may be employed as mixtures thereof. Further, certain cyclic ethers of formula XIV, where b is 3 and the carbocyclic portion of the ring is substituted are resistant to polymerization alone, but copolymerize quite readily with more reactive cyclic ethers. Suitable co-monomers include, for example, 2-methyl-tetrahydrofuran and 3-methyl-tetrahydrofuran. Also, while ethylene oxide may be employed as a co-monomer, ethylene oxide polymers, in the absence of more hydrophobic units, are characteristically too hydrophilic and too excessively absorbs aqueous fluid to be of use in accordance with the instant invention.

In general, the amount of ethoxy units in the polymer backbone of the instant polymeric ophthalmic devices will depend upon the amount of water absorbed by such polymer under use conditions. The polymers for use according to the instant invention absorb more than about 10 % by weight water based upon the total weight of polymer, preferably not more than about 90 % by weight water, more preferably 20 %-75 %, still more preferably 35-55 % by weight water. The absorption amount of water is generally or conveniently measured at about 20°C using distilled water or, if desired, an isotonic solution.

Many polymer diols of the formula HO-A-H are commercially available and include poloxamers having the general formula

$$HO(CH_2CH_2O)_{a'}-(CH(CH_3)CH_2O)_{b'}-(CH_2CH_2O)_{c'}-H$$

wherein b′ has a value between about 16 and 70 and the sum of a′ and c′ is between about 4 and about 100. Examples of such poloxamers, and their average values of a′, b′ and c′, include poloxamer 101 (a′ is 2, b′ is 16, c′ is 2); poloxamer 105 (a′ is 11, b′ is 16, c′ is 11); poloxamer 108 (a′ is 46, b′ is 16, c′ is 46); poloxamer 122 (a′ is 5, b′ is 21, c′ is 5); poloxamer 124 (a′ is 11, b′ is 21, c′ is 11); poloxamer 181 (a′ is 3, b′ is 30, c′ is 3); poloxamer 182 (a′ is 8, b′ is 30, c′ is 8); poloxamer 183 (a′ is 10, b′ is 30, c′ is 10); poloxamer 185 (a′ is 19, b′ is 30, c′ is 19); poloxamer 212 (a′ is 8, b′ is 35, c′ is 8); poloxamer 231 (a′ is 6, b′ is 39, c′ is 6); poloxamer 282 (a′ is 10, b′ is 47, c′ is 10); poloxamer 333 (a′ is 7, b′ is 54, c′ is 7); poloxamer 401 (a′ is 6, b′ is 67, c′ is 6).

Such poloxamers are available, e.g. from BASF Wyandotte under their Pluronic®, brand name.

Polypropylene ether glycols include commercially available products having a molecular weight range between about 400 and about 4,000. Also commercially available are polytetramethylene ether glycols of moderately low molecular weight, generally between about 1,000 and 2,000, and polymers of 1,2 butylene oxide, i.e. polybutyl ether glycol.

As stated above, the polymers for use in the instant invention are those which exhibit a receding contact angle at 20°C of less than 60°, preferably less than 40°, more preferably less than 25°, more preferably less than 15° and most preferably less than 10°. The measurement of such contact angle is conveniently performed using a modified "Wilhelmy Plate" technique, as described, for example, for J.D. Androde, et al. Surface and Interfacial Aspects of Biomedical Polymers, Vol. 1, Surface Chemistry and Physics, Plenum Press, 1985, wherein a specimen sample in the form of a plate of known dimensions is immersed into the wetting solution, pure water, at a slow controlled rate, e.g. at 2 - 20 mm per minute. Poly(hydroxyethylmethacrylate) generally has, under these conditions, a receding contact angle of 39 - 43°.

As mentioned above, the instant polymers for use in the present invention possess a high degree of oxygen permeability. The oxygen permeability, Dk($x10^{-10}$), is measured using a modification of ASTM standard D3985-81 in that (a) there is used 21 % oxygen, i.e. air, instead of 99 - 100 % oxygen, (b) the surface area of sample employed is 0.50 square meters versus 100 square meters and the humidity is controlled to be at 95 -100 % relative humidity instead of 0 % relative humidity. The unit of Dk is [(cm·mm/s)(ml $O_2$/ml·mmHg)].

Typically, conventional fully swollen polyhydroxyethyl methacrylate (HEMA) lenses which are sparingly crosslinked possess a Dk($x10^{-10}$) [(cm·mm/s)(ml $O_2$/ml·mmHg)] value of about 5 - 7.

The oxygen permeability of the instant polymers for use as an ophthalmic device, such as a contact lens, advantageously possess a Dk($x10^{-10}$) value generally greater than 7, preferably greater than about 15, more preferably greater than about 20 and most preferably greater than about 40.

The following examples are for illustrative purposes and are not to be construed as limiting the invention. All parts are by weight unless otherwise specified.

All of the following examples have highly preferred procedures common to each. These common procedures are as follows:

1. All glassware is dried in an oven which is at 150°C for at least 5 - 6 hours.

2. When assembled the reaction system must stay under a constant nitrogen environment.
3. All of the isocyanates used should be freshly distilled.
4. All of the polyglycol material should contain no more than 0.005 % water. For these examples all of the diols are stripped of water using a Pope wipe film still at 65°C and less than 265 Pa.
5. After the reaction glassware is assembled and under a nitrogen atmosphere the set up is flame dried for 20 minutes to ensure that all of the moisture is absent from the system.
6. All of the methylene chloride used in these reactions is distilled into molecular sieves through a 20 mm column packed with glass helices.
7. All temperatures given are degrees Centigrade. Evaporations under reduced pressure are conducted at 2 to 13 kPa if not specified otherwise.

Example 1: 1.0 mole of propylene glycol (previously dried using 4 Å molecular sieves) and 3 % by weight KOH are added to a stainless steel reactor, purged with nitrogen 5 to 7 times and then evacuated to 0 psig for at least 20-30 minutes. During this time period, the reaction mixture is gradually heated to 100°C. The reaction mixture vacuum is broken with a nitrogen sparge to approximately 1-2 psig. Then 4 mole of ethylene oxide is added in a manner so as not to exceed a temperature of 180°C and a pressure of 80 psig. The ethylene oxide is reacted down to 3-4 psig. 11.5 mole of propylene oxide is then added to the reaction mixture in such a manner as not to exceed a temperature of 180°C and a pressure of 80 psig, which is then reacted down to 3-4 psig. The additions of ethylene oxide and propylene oxide, in the same molar ratios, are repeated until the desired molecular weight is achieved. The mixture is then stripped at 100°C to 0 psig to remove any residual oxides, after which the mixture is neutralized and dried. Diol 1 has a molecular weight of 1480 and diol 2 has a molecular weight of 4740. In each of diol 1 and diol 2, the blocks of polyethylene glycol are no longer than 3 ethylene oxide units, while the blocks of polypropylene glycol are no longer than 7 propylene oxide units.

Example 2: To a three neck round bottom flask fitted with an air cooled condensor, dropping funnel, Claisen adaptor, mechanical stirring bar, nitrogen inlet and outlet with the outlet equipped with a desiccant tube is added 250 g (0.0527 mol) of diol 2 of example 1, 350 g of methylene chloride, and 0.45 g (0.0011 mol) of stannous octoate. The reactants are well stirred for 45 minutes. To a dropping funnel is added 15.29 g (0. 1055 mol) of styrene isocyanate and 150 g of methylene chloride. The isocyanate mixture is added over a six to eight hour period dropwise to the diol; checking the reaction mixture occasionally for heat generated in the course of the reaction. It is imperative that the temperature of the reaction not exceed 35°C or generally color will develop in the reaction mixture. If the temperature starts to increase the contents of the reaction mixture should be cooled by the use of an icebath. After three to four hours the completion of the reaction can be followed by IR spectroscopy by the disappearance of the hydroxyl absorption at 3500 $cm^{-1}$. The completion of the reaction can again be followed by IR by observing the disappearance of the NCO absorption at 2270 $cm^{-1}$. The reaction mixture is then stirred for an additional 2 hours. The prepolymer solution is transferred to a single neck flask which is then connected to a rotary evaporator so that the methylene chloride can be removed. The stripping is done at approximately 660 Pa and ambient temperature for the first hour then at 30°C for the last thirty minutes. The prepolymer is cured at 3-5 milliwatts for thirty to ninety minutes.

Example 3: To a three neck round bottom flask fitted with an air cooled condensor, dropping funnel, Claisen adaptor, mechanical stirring bar, nitrogen inlet and outlet with the outlet equipped with a desiccant tube is added 250 g (0.1689 mol) of diol 1 of example 1, 350 g of methylene chloride, and 0.45 g (0.0011 mol) of stannous octoate. The reactants are well stirred for 45 minutes. To a dropping funnel is added 48.99 g (0.3378 mol) of styrene isocyanate and 150 g of methylene chloride. The isocyanate mixture is added over a six to eight hour period dropwise to the diol; checking the reaction mixture occasionally for heat generated in the course of the reaction. It is imperative that the temperature of the reaction not exceed 35°C or generally color will develop in the reaction mixture. If the temperature starts to increase the contents of the reaction mixture should be cooled by the use of an icebath. After three to four hours the completion of the reaction can be followed by IR spectroscopy by the disappearance of the hydroxyl absorption at 3500 $cm^{-1}$. The completion of the reaction can again be followed by IR by observing the disappearance of the NCO absorption at 2270 $cm^{-1}$. The reaction mixture is then stirred for an additional 2 hours. The prepolymer solution is transferred to a single neck flask which is then connected to a rotary evaporator so that the methylene chloride can be removed. The stripping is done at approximately 660 Pa and ambient temperature for the first hour then at 30°C for the last thirty minutes. The prepolymer is cured at 3-5 milliwatts for thirty to ninety minutes.

Example 4: To a three neck round bottom flask fitted with an air cooled condensor, dropping funnel, Claisen adaptor, mechanical stirring bar, nitrogen inlet and outlet with the outlet equipped with a desiccant tube is added 500 g (0.1055 mol) of diol 2 of example 1, 350 g of methylene chloride, and 0.45 g (0.0011 mol) of stannous octoate. The reactants are well stirred for 45 minutes. To a dropping funnel is added 9.18 g (0.0527 mol) of toluene diisocyanate (TDI) and 150 g of methylene chloride. The isocyanate mixture is added over a two to three hour period dropwise to the diol; checking the reaction mixture occasionally for heat generated in the

course of the reaction. It is imperative that the temperature of the reaction not exceed 35°C or generally color will develop in the reaction mixture. If the temperature starts to increase the contents of the reaction mixture should be cooled by the use of an icebath. After three to four hours the completion of the reaction can be followed by IR spectroscopy by the appearance of the isocyanate absorption at 2270 $cm^{-1}$ and the reduction of the hydroxyl peak. To another dropping funnel is added 15.29 g (0.1055 mol) of styrene isocyanate which is slowly added to the reaction mixture when the hydroxyl peak and the isocyanate peak in the IR spectra have disappeared the reaction can be assumed to be complete. After the completion of the reaction 0.40 % Darocur is added and the reaction mixture is then stirred for an additional 2 hours. The prepolymer solution is transferred to a single neck flask which is then connected to a rotary evaporator so that the methylene chloride can be removed. The stripping is done at approximately 660 Pa and ambient temperature for the first hour then at 30°C for the last thirty minutes. The prepolymer is cured at 3-5 milliwatts for thirty to ninety minutes.

Examples 5-7: The procedure of example 4 is followed using the diol specified in the Table below and the amounts of reactants in the Table below.

| | Examples | | | | | |
|---|---|---|---|---|---|---|
| | 2 | 3 | 4 | 5 | 6 | 7 |
| Diol (Type) | 2 | 1 | 2 | 1 | 2 | 1 |
| Diol (g) | 250 | 250 | 500 | 500 | 250 | 250 |
| Diol (mol) | 0.0527 | 0.1689 | 0.1055 | 0.3378 | 0.0527 | 0.1689 |
| Styrene Isocyanate (g) | 15.3 | 48.99 | 15.30 | 48.99 | 5.080 | 16.23 |
| Styrene Isocyanate (mol) | 0.1055 | 0.3378 | 0.1055 | 0.3378 | 0.0351 | 0.1126 |
| Toluene diisocyanate (g) | 0 | 0 | 9.18 | 29.42 | 6.113 | 9.61 |
| Toluene diisocyanate (mol) | 0 | 0 | 0.0527 | 0.1689 | 0.0351 | 0.1126 |
| Ratio of -NCO to OH-groups | 2:1 | 2:1 | 3:2 | 3:2 | 4:3 | 4:3 |
| % water | 60 | 23 | | | | |
| Dk (multiples of HEMA) | 5 | 4 | | | | |

## Claims

### Claims for the following Contracting States : AT, BE, CH, DE, FR, GB, GR, IT, LI, LU, NL, SE

1. A wettable, flexible, oxygen permeable, crosslinked polymer having units of the formula

$$[-A-L-D-]_w \quad (I)$$

wherein each w is 1-8;
each D is -O- or $-NR^7-$ in which $R^7$ is hydrogen, lower alkyl, or phenyl;
each L is independently selected from -B-R-B′-, -BR- or RB′-, wherein each B and B′ is independently selected from

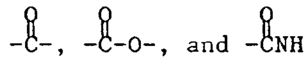

with the carbonyl group thereof being bound to A or D;
each R is a divalent linking group selected from

a) a divalent aliphatic group of up to 25 carbon atoms which may be interrupted by an interrupting unit selected from oxy, carbonyloxy, amino, aminocarbonyl, oxycarbonyl, ureido, oxycarbonylamino, and carbonylamino;

b) a divalent $-(aliphatic)_{alpha}$-5-7 membered cycloaliphatic-$(aliphatic)_{beta}$-group wherein each of alpha and beta is independently 0 or 1, and each of the non cyclic aliphatic groups is independently selected from group a) above, said group b) having up to 25 carbon atoms;

c) a divalent $-(aliphatic)_{alpha}$-aryl-$(aliphatic)_{beta}$-group having 6 to 25 carbon atoms in the aryl portions; and each of the aliphatic groups are independently selected from group a) above and alpha and beta are independently 0 or 1, wherein groups b) and c) can be interrupted in the non-cyclic portions thereof or between the cyclic and non-cyclic portions thereof by an interrupting group as defined in a) above, and groups c) may be further substituted with one or more substituents selected from halogen, $C_{1-4}$ alkyl, and $C_{1-12}$ perhaloalkyl;

and each A is a copolymeric block of polyoxyalkylene, of which at least 30 % of the A groups are selected from

aa) homopolymer blocks of the formula

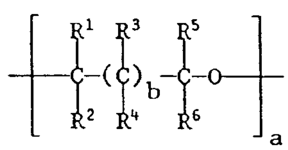

(A)

wherein b is 0 to 4, a is up to 15 and $R^1$-$R^6$ are as defined hereinafter and

ab) copolymer blocks of the formula

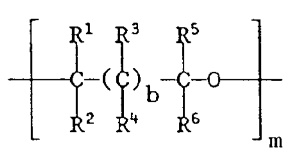

(B)

wherein b is 0 to 4, $R^1$-$R^6$ are as defined hereinafter,
and m is up to 1000 provided that within m, there is no homopolymeric block of more than 15 oxyalkylene units,
the remaining groups being homopolymers or copolymers of the formula

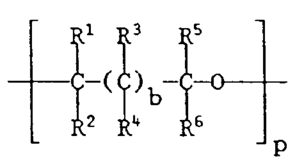

(C)

wherein b is 0 to 4, p is up to 1000 without regard for the size of the homopolymeric sub-blocks within p; the terminal oxygen within the A group being eventually replaced by $-NR^7-$ and wherein each of R1, $R^2$, $R^5$ and $R^6$ is independently selected from the group consisting of hydrogen, an aliphatic, aromatic or heterocyclic radical selected from unsubstituted $C_1$-$C_{16}$ alkyl; substituted $C_1$-$C_{16}$ alkyl; unsubstituted $C_2$-$C_{16}$ alkenyl; and substituted $C_2$-$C_{16}$ alkenyl; wherein the alkyl and alkenyl substituents are independently selected from $C_1$-$C_{16}$ alkoxycarbonyl, $C_2$-$C_{16}$ alkenyloxycarbonyl, fluoro, aryl of up to 10 carbon atoms, $C_1$-$C_8$ alkoxy, $C_2$-$C_6$ alkanoyloxy, aryloxy of up to 10 carbon atoms, $C_3$-$C_6$ alkenoyloxy, aroyloxy of up to 11 carbon atoms, $C_3$-$C_8$ cycloalkyl, $C_3$-$C_8$ cycloalkoxy, $C_3$-$C_8$ cycloalkyl-carbonyloxy, $C_3$-$C_8$ cycloalkoxy-carbonyl, oxacycloalkyl of up to 7 carbon atoms, oxacycloalkoxy of up to 7 carbon atoms, oxacycloalkoxy (up to 7 carbon atoms)-carbonyl, oxacycloalkyl (up to 7 carbon atoms)-carbonyloxy, and aryl (of up to 10 carbon atoms)-oxycarbonyl, each of said alkyl and alkenyl substituents being, in turn, optionally substituted by $C_1$-$C_6$ alkyl, fluoro or a $C_1$-$C_6$ alkoxy provided said last mentioned alkoxy is not bound to a carbon atom already bound to another oxygen atom; $R^1$, $R^2$, $R^5$ and $R^6$ being further independently selected from aryl of up to 10 carbon atoms, $C_3$-$C_8$ cycloalkyl, and oxacycloalkyl of up to 7 carbon atoms, each of which may be unsubstituted or further substituted with a substituent selected from the group of substituents for said alkyl set forth above;
each $R^3$ and $R^4$ are selected from the same group set forth above for $R^1$; and $R^3$ and $R^4$ are further independently selected from $C_1$-$C_{16}$ alkoxycarbonyl, $C_2$-$C_{16}$ alkanoyloxy, $C_2$-$C_{16}$ alkenyloxycarbonyl, and $C_3$-$C_{16}$ alkenoyloxy, each of which may be further substituted by fluoro, aryl of up to 10 carbon atoms, or $C_1$-$C_{16}$ alkoxy, and $R^3$ and $R^4$ are still further independently selected from aryloxy of up to 10 carbon atoms, cycloalkoxy of up to 8 carbon atoms, cycloalkyl (of up to 8 carbon atoms)-carbonyloxy, cycloalkoxy (of up to 8 carbon atoms)-carbonyl, aroyloxy of up to 11 carbon atoms, oxacycloalkoxy of up to 7 carbon atoms, oxacycloalkenyloxy of up to 7 carbon atoms, oxacycloalkoxy (of up to 7 carbon atoms)-carbonyl, oxacycloalkyl (of up to 7 carbon atoms)-carbonyloxy, oxacycloalkenyloxy (of up to 7 carbon atoms)-carbonyl, and aryloxy (of up to 10 carbon atoms)-carbonyl, each of which may be further substituted by fluoro, $C_1$-$C_6$ alkyl or $C_1$-$C_6$ alkoxy, provided that any substituent having an oxygen atom or carbonyl group thereof as its link to the rest of the molecule may not be a substituent on the same carbon atom which is bonded to another oxygen atom, in addition, 2 adjacent groups selected from $R^1$ to $R^6$, together with the atoms to which they are attached may form a 5 - 8 membered cycloalkyl, 5 - 8 membered oxacycloalkyl or bicycloalkyl ring, such that the resulting polymer absorbs more than 10 % by weight water based upon the total weight of polymer.

**2.** The polymer of claim 1 wherein at least 50 % of said -A- groups are selected from formula A and formula B.

**3.** The polymer of claim 1 wherein at least 75 % of said -A- groups are selected from formula A and formula B.

**4.** The polymer of claim 1 wherein all of said -A- groups are selected from formula A and formula B.

**5.** The polymer of claim 1 wherein said -A- groups are selected from formula A and formula C.

**6.** The polymer of claim 1 wherein said -A- groups are selected from formula B and C.

**7.** The polymer of claim 1 wherein each a is no greater than 10.

**8.** The polymer of claim 1 wherein each a is no greater than 7.

**9.** The polymer of claim 1 wherein each a is no greater than 4.

**10.** The polymer of claim 1 wherein each formula B has no homopolymeric subblock having more than 10 oxyalkylene units.

**11.** The polymer of claim 1 wherein each formula B has no homopolymeric subblock having more than 7 oxyalkylene units.

**12.** The polymer of claim 1 wherein each formula B has no homopolymeric subblock having more than 4 oxyalkylene units.

**13.** The polymer of claim 1 characterized in that the units of the formula I are part of a monomer of the formula

$$L'\text{-}D\text{-}[A\text{-}L\text{-}D]_w\text{-}A\text{-}L'' \qquad (III)$$

wherein A and w are as defined in claim 1, provided that not all A groups in any one polymer can be homopolymers of polyethylene glycol or polypropylene glycol;
wherein each D is independently oxygen or $-N(R^7)-$; each $R^7$ is independently selected from hydrogen,

$C_1$-$C_4$ alkyl and phenyl;
wherein each L is independently selected from -BRB′-; wherein B, R and B′ are as defined in claim 1;
wherein L′ is selected from hydrogen, P′-B-$R^x$-B′-, and P′-$R^x$-B′-, wherein each $R^x$ is independently selected from

a) a divalent aliphatic group, preferably alkyl, alkenyl, or alkynyl, of up to 25 carbon atoms which may be interrupted by an interrrupting unit selected from oxy, carbonyloxy, amino, aminocarbonyl, oxycarbonyl, ureido, oxycarbonylamino, and carbonylamino;
b) a divalent 5-7 membered cycloaliphatic group having 5 to 25 carbon atoms;
c) a divalent arylene group having 6 to 25, preferably 7-16 carbon atoms;
d) a divalent aralkyl or alkaryl group having 7 to 25, preferably 8 to 16 carbon atoms;

wherein groups b) and d) can be interrupted in the non-cyclic portions thereof or between the cyclic and non-cyclic portions thereof by an interrupting group as defined in a) above, and groups c) and d) may be further substituted in the aryl portions thereof with one or more substituents selected from halogen, preferably fluorine or chlorine, $C_{1-4}$ alkyl, preferably methyl, and $C_{1-12}$ perhaloalkyl, especially $C_{1-12}$ perfluoralkyl;
with the carbonyl group of B being bound to P′, and P′ is hydrogen, amino, hydroxy, or a moiety containing a crosslinkable group which may be crosslinked when coreacted with a suitable crosslinking agent or when irradiated by actinic radiation;
and L″ is selected from hydrogen, L‴ as defined hereinafter, -B-$R^x$-B′-P′, and -B-$R^x$-P′, wherein $R^x$ and P′ are as defined above except that the carbonyl of B′ instead of B is bound to P′; and L‴ is a terminal monovalent aliphatic, aromatic or cycloaliphatic group of up to 14 carbon atoms.

**14.** The polymer of claim 13 wherein P′ is a vinyl containing moiety.

**15.** The polymer of claim 13 wherein P′ is $H(R^b)C=C(R^a)$- wherein one of $R^a$ and $R^b$ is hydrogen and the other is methyl or hydrogen.

**16.** An ophthalmic device comprising a crosslinked polymer of claim 1.

**17.** An ophthalmic device according to claim 16 characterized in that it comprises a polymer as defined in claim 13.

**18.** An ophthalmic device according to claim 16 characterized in that it is a contact lens.

**19.** An ophthalmic device according to claim 17 characterized in that it is a contact lens.

**20.** A method of correcting a visual defect comprising placing the ophthalmic device of claim 16 in the line of view of an eye having such defect.

**21.** A method according to claim 20 characterized in placing the ophthalmic device of claim 17 in the line of view of an eye having such defect.

**22.** The use of a crosslinked polymer of claim 1 for the manufacture of an ophthalmic device.

**23.** The use according to claim 22 characterized in that the ophthalmic device is a contact lens.

## Claims for the following Contracting State : ES

**1.** A process for the manufacture of a wettable, flexible, oxygen permeable, crosslinked polymer having units of the formula

$$[-A-L-D-]_w \qquad (I)$$

wherein each w is 1-8;
each D is -O- or -$NR^7$- in which $R^7$ is hydrogen, lower alkyl, or phenyl;
each L is independently selected from -B-R-B′-, -BR- or RB′-, wherein each B and B′ is independently selected from

$$-\overset{O}{\overset{\|}{C}}-, \quad -\overset{O}{\overset{\|}{C}}-O-, \quad \text{and} \quad -\overset{O}{\overset{\|}{C}}NH$$

with the carbonyl group thereof being bound to A or D;

each R is a divalent linking group selected from

a) a divalent aliphatic group of up to 25 carbon atoms which may be interrupted by an interrupting unit selected from oxy, carbonyloxy, amino, aminocarbonyl, oxycarbonyl, ureido, oxycarbonylamino, and carbonylamino;

b) a divalent -(aliphatic)$_{alpha}$-5-7 membered cycloaliphatic-(aliphatic)$_{beta}$-group wherein each of alpha and beta is independently 0 or 1, and each of the non cyclic aliphatic groups is independently selected from group a) above, said group b) having up to 25 carbon atoms;

c) a divalent -(aliphatic)$_{alpha}$-aryl-(aliphatic)$_{beta}$-group having 6 to 25 carbon atoms in the aryl portions; and each of the aliphatic groups are independently selected from group a) above and alpha and beta are independently 0 or 1, wherein groups b) and c) can be interrupted in the non-cyclic portions thereof or between the cyclic and non-cyclic portions thereof by an interrupting group as defined in a) above, and groups c) may be further substituted with one or more substituents selected from halogen, $C_{1-4}$ alkyl, and $C_{1-12}$ perhaloalkyl;

and each A is a copolymeric block of polyoxyalkylene, of which at least 30 % of the A groups are selected from

aa) homopolymer blocks of the formula

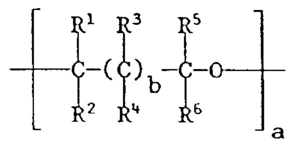

(A)

wherein b is 0 to 4, a is up to 15 and $R^1$-$R^6$ are as defined hereinafter and

ab) copolymer blocks of the formula

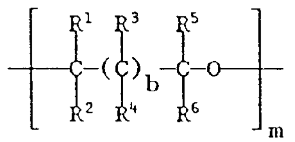

(B)

wherein b is 0 to 4, $R^1$-$R^6$ are as defined hereinafter,

and m is up to 1000 provided that within m, there is no homopolymeric block of more than 15 oxyalkylene units,

the remaining groups being homopolymers or copolymers of the formula

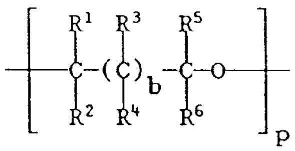

(C)

wherein b is 0 to 4, p is up to 1000 without regard for the size of the homopolymeric sub-blocks within p; the terminal oxygen within the A group being eventually replaced by -$NR^7$- and wherein each of $R^1$, $R^2$, $R^5$ and $R^6$ is independently selected from the group consisting of hydrogen, an aliphatic, aromatic or heterocyclic radical selected from unsubstituted $C_1$-$C_{16}$ alkyl; substituted $C_1$-$C_{16}$ alkyl; unsubstituted $C_2$-$C_{16}$ alkenyl; and substituted $C_2$-$C_{16}$ alkenyl; wherein the alkyl and alkenyl substituents are independently selected from $C_1$-$C_{16}$ alkoxycarbonyl, $C_2$-$C_{16}$ alkenyloxycarbonyl, fluoro, aryl of up to 10 carbon atoms, $C_1$-$C_8$ alkoxy, $C_2$-$C_6$ alkanoyloxy, aryloxy of up to 10 carbon atoms, $C_3$-$C_6$ alkenoyloxy, aroyloxy of up to 11 carbon atoms, $C_3$-$C_8$ cycloalkyl, $C_3$-$C_8$ cycloalkoxy, $C_3$-$C_8$ cycloalkyl-carbonyloxy, $C_3$-$C_8$ cycloalkoxy-carbonyl, oxacycloalkyl of up to 7 carbon atoms, oxacycloalkoxy of up to 7 carbon atoms, oxacycloalkoxy (up to 7 carbon atoms)-carbonyl, oxacycloalkyl (up to 7 carbon atoms)-carbonyloxy, and aryl (of up to 10 carbon atoms)-oxycarbonyl, each of said alkyl and alkenyl substituents being, in turn, optionally substituted by $C_1$-$C_6$ alkyl, fluoro or a $C_1$-$C_6$ alkoxy provided said last mentioned alkoxy is not bound to a carbon atom already bound to another oxygen atom; $R^1$, $R^2$, $R^5$ and $R^6$ being further independently selected from aryl of up to 10 carbon atoms, $C_3$-$C_8$ cycloalkyl, and oxacycloalkyl of up to 7 carbon atoms, each of which may be unsubstituted or further substituted with a substituent selected from the group of substituents for said alkyl set forth above;

each $R^3$ and $R^4$ are selected from the same group set forth above for $R^1$; and $R^3$ and $R^4$ are further independently selected from $C_1$-$C_{16}$ alkoxycarbonyl, $C_2$-$C_{16}$ alkanoyloxy, $C_2$-$C_{16}$ alkenyloxycarbonyl, and $C_3$-$C_{16}$ alkenoyloxy, each of which may be further substituted by fluoro, aryl of up to 10 carbon atoms, or $C_1$-$C_{16}$ alkoxy, and $R^3$ and $R^4$ are still further independently selected from aryloxy of up to 10 carbon atoms, cycloalkoxy of up to 8 carbon atoms, cycloalkyl (of up to 8 carbon atoms)-carbonyloxy, cycloalkoxy (of up to 8 carbon atoms)-carbonyl, aroyloxy of up to 11 carbon atoms, oxacycloalkoxy of up to 7 carbon atoms, oxacycloalkenyloxy of up to 7 carbon atoms, oxacycloalkoxy (of up to 7 carbon atoms)-carbonyl, oxacycloalkyl (of up to 7 carbon atoms)-carbonyloxy, oxacycloalkenyloxy (of up to 7 carbon atoms)-carbonyl, and aryloxy (of up to 10 carbon atoms)-carbonyl, each of which may be further substituted by fluoro, $C_1$-$C_6$ alkyl or $C_1$-$C_6$ alkoxy, provided that any substituent having an oxygen atom or carbonyl group thereof as its link to the rest of the molecule may not be a substituent on the same carbon atom which is bonded to another oxygen atom, in addition, 2 adjacent groups selected from $R^1$ to $R^6$, together with the atoms to which they are attached may form a 5 - 8 membered cycloalkyl, 5 - 8 membered oxacycloalkyl or bicycloalkyl ring, such that the resulting polymer absorbs more than 10 % by weight water based upon the total weight of polymer, by crosslinking in a manner known per se.

2. The process of claim 1 wherein at least 50 % of said -A- groups are selected from formula A and formula B.

3. The process of claim 1 wherein at least 75 % of said -A- groups are selected from formula A and formula B.

4. The process of claim 1 wherein all of said -A- groups are selected from formula A and formula B.

5. The process of claim 1 wherein said -A- groups are selected from formula A and formula C.

6. The process of claim 1 wherein said -A- groups are selected from formula B and C.

7. The process of claim 1 wherein each a is no greater than 10.

8. The process of claim 1 wherein each a is no greater than 7.

9. The process of claim 1 wherein each a is no greater than 4.

10. The process of claim 1 wherein each formula B has no homopolymeric subblock having more than 10 oxyalkylene units.

11. The process of claim 1 wherein each formula B has no homopolymeric subblock having more than 7 oxyalkylene units.

12. The process of claim 1 wherein each formula B has no homopolymeric subblock having more than 4 oxyalkylene units.

13. The process of claim 1 characterized in that the units of the formula I are part of a monomer of the formula

$$L'\text{-}D\text{-}[A\text{-}L\text{-}D]_w\text{-}A\text{-}L'' \quad (III)$$

wherein A and w are as defined in claim 1, provided that not all A groups in any one polymer can be homopolymers of polyethylene glycol or polypropylene glycol;
wherein each D is independently oxygen or -N($R^7$)-; each $R^7$ is independently selected from hydrogen, $C_1$-$C_4$ alkyl and phenyl;
wherein each L is independently selected from -BRB′-; wherein B, R and B′ are as defined in claim 1;
wherein L′ is selected from hydrogen, P′-B-$R^x$-B′-, and P′-$R^x$-B′-, wherein each $R^x$ is independently selected from

a) a divalent aliphatic group, preferably alkyl, alkenyl, or alkynyl, of up to 25 carbon atoms which may be interrupted by an interrrupting unit selected from oxy, carbonyloxy, amino, aminocarbonyl, oxycarbonyl, ureido, oxycarbonylamino, and carbonylamino;
b) a divalent 5-7 membered cycloaliphatic group having 5 to 25 carbon atoms;
c) a divalent arylene group having 6 to 25, preferably 7-16 carbon atoms;
d) a divalent aralkyl or alkaryl group having 7 to 25, preferably 8 to 16 carbon atoms;

wherein groups b) and d) can be interrupted in the non-cyclic portions thereof or between the cyclic and non-cyclic portions thereof by an interrupting group as defined in a) above, and groups c) and d) may be further substituted in the aryl portions thereof with one or more substituents selected from halogen, preferably fluorine or chlorine, $C_{1-4}$ alkyl, preferably methyl, and $C_{1-12}$ perhaloalkyl, especially $C_{1-12}$ perfluoralkyl;

with the carbonyl group of B being bound to P′, and P′ is hydrogen, amino, hydroxy, or a moiety containing a crosslinkable group which may be crosslinked when coreacted with a suitable crosslinking agent or when irradiated by actinic radiation;
and L″ is selected from hydrogen, L‴ as defined hereinafter, -B-$R^x$-B′-P′, and -B-$R^x$-P′, wherein $R^x$ and P′ are as defined above except that the carbonyl of B′ instead of B is bound to P′; and L‴ is a terminal monovalent aliphatic, aromatic or cycloaliphatic group of up to 14 carbon atoms.

**14.** The process of claim 13 wherein P′ is a vinyl containing moiety.

**15.** The process of claim 13 wherein P′ is $H(R^b)C=C(R^a)$- wherein one of $R^a$ and $R^b$ is hydrogen and the other is methyl or hydrogen.

**16.** An ophthalmic device comprising a crosslinked polymer of claim 1.

**17.** An ophthalmic device according to claim 16 characterized in that it comprises a polymer as defined in claim 13.

**18.** An ophthalmic device according to claim 16 characterized in that it is a contact lens.

**19.** An ophthalmic device according to claim 17 characterized in that it is a contact lens.

**20.** A method of correcting a visual defect comprising placing the ophthalmic device of claim 16 in the line of view of an eye having such defect.

**21.** A method according to claim 20 characterized in placing the ophthalmic device of claim 17 in the line of view of an eye having such defect.

**22.** The use of a crosslinked polymer of claim 1 for the manufacture of an ophthalmic device.

**23.** The use according to claim 22 characterized in that the ophthalmic device is a contact lens.

## Patentansprüche

### Patentansprüche für folgende Vertragsstaaten : AT, BE, CH, DE, FR, GB, GR, IT, LI, LU, NL, SE

**1.** Benetzbares, flexibles, sauerstoffdurchlässiges, vernetztes Polymeres, das Einheiten der Formel

$$[-A-L-D-]_w \qquad (I)$$

enthält, worin jeweils w die Bedeutung 1 bis 8 hat;
jedes D ist -O- oder -$NR^7$, worin $R^7$ Wasserstoff, Niederalkyl oder Phenyl bedeutet;
jedes L ist unabhängig ausgewählt unter -B-R-B′-, -BR- oder RB′-, worin jedes B und B′ unabhängig ausgewählt ist unter

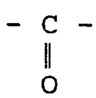 , 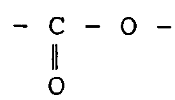 und 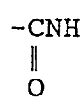

worin deren Carbonylgruppe an A oder D gebunden ist;
jedes R ist eine zweiwertige Verknüpfungsgruppe, ausgewählt unter

a) einer zweiwertigen aliphatischen Gruppe mit bis zu 25 Kohlenstoffatomen, die durch eine Unterbrechungseinheit unterbrochen sein kann, ausgewählt unter Oxy, Carbonyloxy, Amino, Aminocarbonyl, Oxycarbonyl, Ureido, Oxycarbonylamino und Carbonylamino;
b) einer zweiwertigen -$(\text{aliphatisch})_{\text{alpha}}$-5-bis 7-gliedrigcycloaliphatisch-$(\text{aliphatisch})_{\text{beta}}$-Gruppe, worin jeweils alpha und beta unabhängig o oder 1 ist, und jede der nichtcyclischen aliphatischen Gruppen ist unabhängig ausgewählt unter der Gruppe a) oben, der genannten Gruppe b) mit bis zu 25 Kohlenstoffatomen;
c) einer zweiwertigen -$(\text{aliphatisch})_{\text{alpha}}$-Aryl-$(\text{aliphatisch})_{\text{beta}}$-Gruppe mit 6 bis 25 Kohlenstoffatomen in den Arylteilen; und jede der aliphatischen Gruppen ist unabhängig ausgewählt aus der Gruppe a)

oben, und alpha und beta sind unabhängig 0 oder 1, wobei die Gruppen b) und c) in deren nichtcyclischen Teilen oder zwischen deren cyclischen und nichtcyclischen Teilen unterbrochen sein können durch eine Unterbrechungsgruppe wie in a) oben definiert, und die Gruppen c) können weiterhin mit einem oder mehreren Substituenten substituiert sein, ausgewählt unter Halogen, $C_1$-$C_4$-Alkyl und $C_1$-$C_{12}$-Perhaloalkyl, und jedes A ist ein Copolymerblock von Polyoxyalkylen, von dem wenigstens 30 % der A-Gruppen ausgewählt sind unter

aa) Homopolymerblöcken der Formel

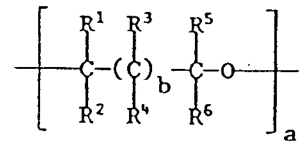

(A)

worin b die Bedeutung 0 bis 4 hat, a ist bis zu 15, und $R^1$ bis $R^6$ sind wie nachfolgend definiert, und

ab) Copolymerblöcken der Formel

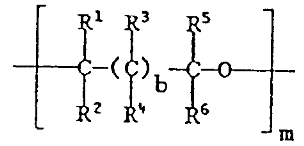

(B)

worin b die Bedeutung 0 bis 4 hat, $R^1$ bis $R^6$ sind wie nachfolgend definiert,
und m ist bis zu 1000, mit der Maßgabe, daß es innerhalb von m keinen Homopolymerblock von mehr als 15 Oxyalkylen-Einheiten gibt,
wobei die restlichen Gruppen Homopolymere oder Copolymere der Formel

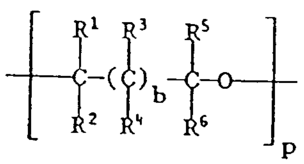

(C)

sind, worin b die Bedeutung 0 bis 4 hat, p ist bis zu 1000 ohne Rücksicht auf die Größe der homopolymeren Unterblöcke innerhalb von p; der terminale Sauerstoff innerhalb der A-Gruppe ist eventuell ersetzt durch $-NR^7$-, und worin jeweils $R^1$, $R^2$, $R^5$ und $R^6$ unabhängig ausgewählt sind unter der Gruppe, die aus Wasserstoff, einem aliphatischen, aromatischen oder heterocyclischen Rest besteht, ausgewählt unter unsubstituiertem $C_1$-$C_{16}$-Alkyl, substituiertem $C_1$-$C_{16}$-Alkyl; unsubstituiertem $C_2$-$C_{16}$-Alkenyl; und substituiertem $C_2$-$C_{16}$-Alkenyl; worin die Alkyl- und Alkenyl-Substituenten unabhängig ausgewählt sind unter $C_1$-$C_{16}$-Alkoxycarbonyl, $C_2$-$C_{16}$-Alkenyloxycarbonyl, Fluor, Aryl mit bis zu 10 Kohlenstoffatomen, $C_1$-$C_8$-Alkoxy, $C_2$-$C_6$-Alkanoyloxy, Aryloxy mit bis zu 10 Kohlenstoffatomen, $C_3$-$C_6$-Alkenoyloxy, Aroyloxy mit bis zu 11 Kohlenstoffatomen, $C_3$-$C_8$-Cycloalkyl, $C_3$-$C_8$-Cycloalkoxy, $C_3$-$C_8$-Cycloalkyl-carbonyloxy, $C_3$-$C_8$-Cycloalkoxy-carbonyl, Oxacycloalkyl mit bis zu 7 Kohlenstoffatomen, Oxacycloalkoxy mit bis zu 7 Kohlenstoffatomen, Oxacycloalkoxy(bis zu 7 Kohlenstoffatome)-carbonyl, Oxacycloalkyl(bis zu 7 Kohlenstoffatome)-carbonyloxy und Aryl(bis zu 10 Kohlenstoffatome)-oxycarbonyl, wobei jeder der Alkyl- und Alkenyl-Substituenten wiederum gegebenenfalls substituiert ist durch $C_1$-$C_6$-Alkyl, Fluor oder $C_1$-$C_6$-Alkoxy, mit der Maßgabe, daß das zuletzt genannte Alkoxy nicht an ein Kohlenstoffatom gebunden ist, das bereits an ein anderes Sauerstoffatom gebunden ist; $R^1$, $R^2$, $R^5$ und $R^6$ sind weiterhin unabhängig ausgewählt unter Aryl mit bis zu 10 Kohlenstoffatomen, $C_3$-$C_8$-Cycloalkyl und Oxacycloalkyl mit bis zu 7 Kohlenstoffatomen, von denen jedes unsubstituiert sein kann oder weiterhin mit einem Substituenten substituiert sein kann, ausgewählt aus der Gruppe, die aus den Substituenten für das obige Alkyl besteht;
jedes $R^3$ und $R^4$ ist ausgewählt aus der gleichen Gruppe, die oben für $R^1$ genannt ist; und $R^3$ und $R^4$ sind weiterhin unabhängig ausgewählt unter $C_1$-$C_{16}$-Alkoxycarbonyl, $C_2$-$C_{16}$-Alkanoyloxy, $C_2$-$C_{16}$-Alkenyloxycarbonyl und $C_3$-$C_{16}$-Alkenoyloxy, von denen jedes weiterhin substituiert sein kann durch Fluor, Aryl mit bis zu 10 Kohlenstoffatomen oder $C_1$-$C_{16}$-Alkoxy, und $R^3$ und $R^4$ sind weiterhin unabhängig ausgewählt unter Aryloxy mit bis zu 10 Kohlenstoffatomen, Cycloalkyloxy mit bis zu 8 Kohlenstoffatomen, Cycloalkyl(bis zu 8 Kohlenstoffatome)-carbonyloxy, Cycloalkoxy(bis zu 8 Kohlenstoffatome)-carbonyl, Aroyloxy mit bis zu 11 Kohlenstoffatomen, Oxacycloalkoxy mit bis zu 7 Kohlenstoffatomen, Oxacycloalkenyloxy mit bis zu 7 Kohlenstoffatomen,

Oxacycloalkoxy(bis zu 7 Kohlenstoffatome)-carbonyl, Oxacycloalkyl(bis zu 7 Kohlenstoffatome)-carbonyloxy, Oxacycloalkenyloxy(bis zu 7 Kohlenstoffatome)-carbonyl und Aryloxy(bis zu 10 Kohlenstoffatome)-carbonyl, von denen jedes weiterhin substituiert sein kann durch Fluor, $C_1$-$C_6$-Alkyl oder $C_1$-$C_6$-Alkoxy, mit der Maßgabe, daß ein beliebiger Substituent, der ein Sauerstoffatom oder eine Carbonylgruppe davon als dessen Bindung an den Rest des Moleküls hat, kein Substituent an dem gleichen Kohlenstoffatom sein kann, das an ein anderes Sauerstoffatom gebunden ist, und wobei zusätzlich zwei benachbarte Gruppen, ausgewählt unter $R^1$ bis $R^6$, zusammen mit den Atomen, an die sie gebunden sind, einen 5- bis 8-gliedrigen Cycloalkylring, einen 5- bis 8-gliedrigen Oxacycloalkylring oder einen Bicycloalkylring bilden können, so daß das erhaltene Polymere mehr als 10 Gewichts-% Wasser absorbiert, bezogen auf das Gesamtgewicht des Polymeren.

**2.** Polymeres nach Anspruch 1, worin wenigstens 50 % der -A-Gruppen ausgewählt sind unter Formel A und B.

**3.** Polymer nach Anspruch 1, worin wenigstens 75 % der -A-Gruppen ausgewählt sind unter Formel A und B.

**4.** Polymer nach Anspruch 1, worin alle -A-Gruppen ausgewählt sind unter Formel A und Formel B.

**5.** Polymer nach Anspruch 1, worin die -A-Gruppen ausgewählt sind unter Formel A und Formel C.

**6.** Polymer nach Anspruch 1, worin die -A-Gruppen ausgewählt sind unter Formel B und C.

**7.** Polymer nach Anspruch 1, worin jedes a nicht größer als 10 ist.

**8.** Polymer nach Anspruch 1, worin jedes a nicht größer als 7 ist.

**9.** Polymer nach Anspruch 1, worin jedes a nicht größer als 4 ist.

**10.** Polymer nach Anspruch 1, worin jede Formel D keinen homopolymeren Unterblock mit mehr als 10 Oxyalkylen-Einheiten hat.

**11.** Polymer nach Anspruch 1, worin jede Formel B keinen homopolymeren Unterblock mit mehr als 7 Oxyalkylen-Einheiten hat.

**12.** Polymer nach Anspruch 1, worin jede Formel B keinen homopolymeren Unterblock mit mehr als 4 Oxyalkylen-Einheiten hat.

**13.** Polymer nach Anspruch 1, dadurch gekennzeichnet, daß die Einheiten der Formel I Teil eines Monomeren der Formel

$$L'\text{-}D\text{-}[A\text{-}L\text{-}D]_w\text{-}A\text{-}L'' \qquad (III)$$

sind, worin A und w wie in Anspruch 1 definiert sind, mit der Maßgabe, daß nicht alle A-Gruppen in irgendeinem Polymeren Homopolymere von Polyethylenglycol oder Polypropylenglycol sein können; worin jedes D unabhängig Sauerstoff oder -N($R^7$)- ist; jedes $R^7$ ist unabhängig ausgewählt unter Wasserstoff, $C_1$-$C_4$-Alkyl und Phenyl;
worin jedes L unabhängig ausgewählt ist unter -BRB′-; worin B, R und B′ wie in Anspruch 1 definiert sind; worin L′ ausgewählt ist unter Wasserstoff, P′-B-Rx-B′- und P′-$R^x$-B′-, worin jedes $R^x$ unabhängig ausgewählt ist unter

a) einer zweiwertigen aliphatischen Gruppe, vorzugsweise Alkyl, Alkenyl oder Alkinyl mit bis zu 25 Kohlenstoffatomen, die unterbrochen sein kann durch eine Unterbrechungseinheit, ausgewählt unter Oxy, Carbonyloxy, Amino, Aminocarbonyl, Oxycarbonyl, Ureido, Oxycarbonylamino und Carbonylamino;
b) einer zweiwertigen 5- bis 7-gliedrigen cycloaliphatischen Gruppe mit 5 bis 25 Kohlenstoffatomen;
c) einer zweiwertigen Arylengruppe mit 6 bis 25, vorzugsweise 7 bis 16 Kohlenstoffatomen;
d) einer zweiwertigen Aralkyl- oder Alkaryl-Gruppe mit 7 bis 25, vorzugsweise 8 bis 16 Kohlenstoffatomen;

worin die Gruppen b) und d) in ihren nichtcyclischen Teilen oder zwischen den cyclischen und nichtcyclischen Teilen unterbrochen sein können durch eine Unterbrechungseinheit, wie sie in a) definiert ist, und die Gruppen c) und d) können weiterhin substituiert sein in ihren Arylteilen mit einem oder mehreren Substituenten, ausgewählt unter Halogen, vorzugsweise Fluor oder Chlor, $C_1$-$C_4$-Alkyl, vorzugsweise Methyl, und $C_1$-$C_{12}$-Perhaloalkyl, insbesondere $C_1$-$C_{12}$-Perfluoralkyl;
mit der Carbonylgruppe von B sind sie gebunden an P′, und P′ ist Wasserstoff, Amino, Hydroxy oder ein Abschnitt, der eine vernetzbare Gruppe enthält, die vernetzt werden kann, wenn sie mit einem geeigneten Vernetzungsmittel coreagiert oder wenn sie durch aktinisches Licht bestrahlt wird;

und L″ ist ausgewählt unter Wasserstoff, L‴ wie nachfolgend definiert, -B-$R^x$-B′-P′ und -B-$R^x$-P′, worin $R^x$ und P′ wie oben definiert sind, mit Ausnahme dessen, daß das Carbonyl von B′ anstelle von B an P′ gebunden ist; und L‴ ist eine terminale einwertige aliphatische, aromatische oder cycloaliphatische Gruppe mit bis zu 14 Kohlenstoffatomen.

**14.** Polymer nach Anspruch 13, worin P′ ein Vinyl-enthaltender Teil ist.

**15.** Polymer nach Anspruch 13, worin P′ die Bedeutung $H(R^b)C = C(R^a)$- hat, worin einer von $R^a$ und $R^b$ Wasserstoff und der andere Methyl oder Wasserstoff ist.

**16.** Ophthalmische Einrichtung, die ein vernetztes Polymer von Anspruch 1 umfaßt.

**17.** Ophthalmische Einrichtung nach Anspruch 16, dadurch gekennzeichnet, daß sie ein Polymer umfaßt wie in Anspruch 13 definiert.

**18.** Ophthalmische Einrichtung nach Anspruch 16, dadurch gekennzeichnet, daß sie eine Kontaktlinse ist.

**19.** Ophthalmische Einrichtung nach Anspruch 17, dadurch gekennzeichnet, daß sie eine Kontaktlinse ist.

**20.** Verfahren zur Korrektur eines Sehfehlers, gekennzeichnet durch die Anordnung der ophthalmischen Einrichtung von Anspruch 16 in die Blicklinie eines Auges, das einen solchen Fehler hat.

**21.** Verfahren nach Anspruch 20, gekennzeichnet durch Anordnung der ophthalmischen Einrichtung von Anspruch 17 in der Blicklinie eines Auges, das einen solchen Fehler hat.

**22.** Verwendung eines vernetzten Polymeren von Beispiel 1 zur Herstellung einer ophthalmischen Einrichtung.

**23.** Verwendung nach Anspruch 22, dadurch gekennzeichnet, daß die ophthalmische Einrichtung eine Kontaktlinse ist.

**Patentansprüche für folgenden Vertragsstaat : ES**

**1.** Verfahren zur Herstellung eines benetzbaren, flexiblen, sauerstoffdurchlässigen, vernetzten Polymeren, das Einheiten der Formel

$$[- A - L - D -]_w \qquad (I)$$

enthält, worin jeweils w die Bedeutung 1 bis 8 hat;
jedes D ist -O- oder -$NR^7$, worin $R^7$ Wasserstoff, Niederalkyl oder Phenyl bedeutet;
jedes L ist unabhängig ausgewählt unter -B-R-B′-, -BR- oder RB′-, worin jedes B und B′ unabhängig ausgewählt ist unter

$$-\underset{\underset{O}{\|}}{C}-, \quad -\underset{\underset{O}{\|}}{C}-O- \quad \text{und} \quad -\underset{\underset{O}{\|}}{C}NH$$

worin deren Carbonylgruppe an A oder D gebunden ist;
jedes R ist eine zweiwertige Verknüpfungsgruppe, ausgewählt unter

a) einer zweiwertigen aliphatischen Gruppe mit bis zu 25 Kohlenstoffatomen, die durch eine Unterbrechungseinheit unterbrochen sein kann, ausgewählt unter Oxy, Carbonyloxy, Amino, Aminocarbonyl, Oxycarbonyl, Ureido, Oxycarbonylamino und Carbonylamino;

b) einer zweiwertigen -$(\text{aliphatisch})_{\text{alpha}}$-5-bis 7-gliedrigcycloaliphatisch-$(\text{aliphatisch})_{\text{beta}}$-Gruppe, worin jeweils alpha und beta unabhängig 0 oder 1 ist, und jede der nichtcyclischen aliphatischen Gruppen ist unabhängig ausgewählt unter der Gruppe a) oben, der genannten Gruppe b) mit bis zu 25 Kohlenstoffatomen;

c) einer zweiwertigen -$(\text{aliphatisch})_{\text{alpha}}$-Aryl-$(\text{aliphatisch})_{\text{beta}}$-Gruppe mit 6 bis 25 Kohlenstoffatomen in den Arylteilen; und jede der aliphatischen Gruppen ist unabhängig ausgewählt aus der Gruppe a) oben, und alpha und beta sind unabhängig 0 oder 1, wobei die Gruppen b) und c) in deren nichtcyclischen Teilen oder zwischen deren cyclischen und nichtcyclischen Teilen unterbrochen sein können durch eine Unterbrechungsgruppe wie in a) oben definiert, und die Gruppen c) können weiterhin mit

einem oder mehreren Substituenten substituiert sein, ausgewählt unter Halogen, $C_1$-$C_4$-Alkyl und $C_1$-$C_{12}$-Perhaloalkyl;
und jedes A ist ein Copolymerblock von Polyoxyalkylen, von dem wenigstens 30 % der A-Gruppen ausgewählt sind unter

aa) Homopolymerblöcken der Formel

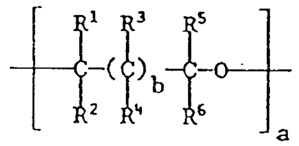

(A)

worin b die Bedeutung 0 bis 4 hat, a ist bis zu 15, und $R^1$ bis $R^6$ sind wie nachfolgend definiert, und

ab) Copolymerblöcken der Formel

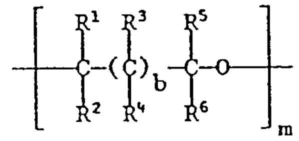

(B)

worin b die Bedeutung 0 bis 4 hat, $R^1$ bis $R^6$ sind wie nachfolgend definiert,
und m ist bis zu 1000, mit der Maßgabe, daß es innerhalb von m keinen Homopolymerblock von mehr als 15 Oxyalkylen-Einheiten gibt,
wobei die restlichen Gruppen Homopolymere oder Copolymere der Formel

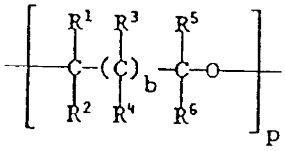

(C)

sind, worin b die Bedeutung 0 bis 4 hat, p ist bis zu 1000 ohne Rücksicht auf die Größe der homopolymeren Unterblöcke innerhalb von p; der terminale Sauerstoff innerhalb der A-Gruppe ist eventuell ersetzt durch $-NR^7-$, und worin jeweils $R^1$, $R^2$, $R^5$ und $R^6$ unabhängig ausgewählt sind unter der Gruppe, die aus Wasserstoff, einem aliphatischen, aromatischen oder heterocyclischen Rest besteht, ausgewählt unter unsubstituiertem $C_1$-$C_{16}$-Alkyl; substituiertem $C_1$-$C_{16}$-Alkyl; unsubstituiertem $C_2$-$C_{16}$-Alkenyl; und substituiertem $C_2$-$C_{16}$-Alkenyl; worin die Alkyl- und Alkenyl-Substituenten unabhängig ausgewählt sind unter $C_1$-$C_{16}$-Alkoxycarbonyl, $C_2$-$C_{16}$-Alkenyloxycarbonyl, Fluor, Aryl mit bis zu 10 Kohlenstoffatomen, $C_1$-$C_8$-Alkoxy, $C_2$-$C_6$-Alkanoyloxy, Aryloxy mit bis zu 10 Kohlenstoffatomen, $C_3$-$C_6$-Alkenoyloxy, Aroyloxy mit bis zu 11 Kohlenstoffatomen, $C_3$-$C_8$-Cycloalkyl, $C_3$-$C_8$-Cycloalkoxy, $C_3$-$C_8$-Cycloalkyl-carbonyloxy, $C_3$-$C_8$-Cycloalkoxy-carbonyl, Oxacycloalkyl mit bis zu 7 Kohlenstoffatomen, Oxacycloalkoxy mit bis zu 7 Kohlenstoffatomen, Oxacycloalkoxy(bis zu 7 Kohlenstoffatome)-carbonyl, Oxacycloalkyl(mit bis zu 7 Kohlenstoffatome)-carbonyloxy und Aryl(bis zu 10 Kohlenstoffatome)-oxycarbonyl, wobei jeder der Alkyl- und Alkenyl-Substituenten wiederum gegebenenfalls substituiert ist durch $C_1$-$C_6$-Alkyl, Fluor oder $C_1$-$C_6$-Alkoxy, mit der Maßgabe, daß das zuletzt genannte Alkoxy nicht an ein Kohlenstoffatom gebunden ist, das bereits an ein anderes Sauerstoffatom gebunden ist; $R^1$, $R^2$, $R^5$ und $R^6$ sind weiterhin unabhängig ausgewählt unter Aryl mit bis zu 10 Kohlenstoffatomen, $C_3$-$C_8$-Cycloalkyl und Oxacycloalkyl mit bis zu 7 Kohlenstoffatomen, von denen jedes unsubstituiert sein kann oder weiterhin mit einem Substituenten substituiert sein kann, ausgewählt aus der Gruppe, die aus den Substituenten für das obige Alkyl besteht;
jedes $R^3$ und $R^4$ ist ausgewählt aus der gleichen Gruppe, die oben für $R^1$ genannt ist; und $R^3$ und $R^4$ sind weiterhin unabhängig ausgewählt unter $C_1$-$C_{16}$-Alkoxycarbonyl, $C_2$-$C_{16}$-Alkanoyloxy, $C_2$-$C_{16}$-Alkenyloxycarbonyl und $C_3$-$C_{16}$-Alkenoyloxy, von denen jedes weiterhin substituiert sein kann durch Fluor, Aryl mit bis zu 10 Kohlenstoffatomen oder $C_1$-$C_{16}$-Alkoxy, und $R^3$ und $R^4$ sind weiterhin unabhängig ausgewählt unter Aryloxy mit bis zu 10 Kohlenstoffatomen, Cycloalkyloxy mit bis zu 8 Kohlenstoffatomen, Cycloalkyl(bis zu 8 Kohlenstoffatome)-carbonyloxy, Cycloalkoxy(bis zu 8 Kohlenstoffatome)-carbonyl, Aroyloxy mit bis zu 11 Kohlenstoffatomen, Oxacycloalkoxy mit bis zu 7 Kohlenstoffatomen, Oxacycloalkenyloxy mit bis zu 7 Kohlenstoffatomen, Oxacycloalkoxy(bis zu 7 Kohlenstoffatome)-carbonyl, Oxacycloalkyl(bis zu 7 Kohlenstoffatome)-carbonyloxy,

Oxacycloalkenyloxy(bis zu 7 Kohlenstoffatome)-carbonyl und Aryloxy(bis zu 10 Kohlenstoffatome)-carbonyl, von denen jedes weiterhin substituiert sein kann durch Fluor, $C_1$-$C_6$-Alkyl oder $C_1$-$C_6$-Alkoxy, mit der Maßgabe, daß ein beliebiger Substituent, der ein Sauerstoffatom oder eine Carbonylgruppe davon als dessen Bindung an den Rest des Moleküls hat, kein Substituent an dem gleichen Kohlenstoffatom sein kann, das an ein anderes Sauerstoffatom gebunden ist, und wobei zusätzlich zwei benachbarte Gruppen, ausgewählt unter $R^1$ bis $R^6$, zusammen mit den Atomen, an die sie gebunden sind, einen 5- bis 8-gliedrigen Cycloalkylring, einen 5- bis 8-gliedrigen Oxacycloalkylring oder einen Bicycloalkylring bilden können, so daß das erhaltene Polymere mehr als 10 Gewichts-% Wasser absorbiert, bezogen auf das Gesamtgewicht des Polymeren,
durch Vernetzung in an sich bekannter Weise.

2. Verfahren nach Anspruch 1, worin wenigstens 50 % der -A-Gruppen ausgewählt sind unter Formel A und B.

3. Verfahren nach Anspruch 1, worin wenigstens 75 % der -A-Gruppen ausgewählt sind unter Formel A und B.

4. Verfahren nach Anspruch 1, worin alle -A-Gruppen ausgewählt sind unter Formel A und Formel B.

5. Verfahren nach Anspruch 1, worin die -A-Gruppen ausgewählt sind unter Formel A und Formel C.

6. Verfahren nach Anspruch 1, worin die -A-Gruppen ausgewählt sind unter Formel B und C.

7. Verfahren nach Anspruch 1, worin jedes a nicht größer als 10 ist.

8. Verfahren nach Anspruch 1, worin jedes a nicht größer als 7 ist.

9. Verfahren nach Anspruch 1, worin jedes a nicht größer als 4 ist.

10. Verfahren nach Anspruch 1, worin jede Formel D keinen homopolymeren Unterblock mit mehr als 10 Oxyalkylen-Einheiten hat.

11. Verfahren nach Anspruch 1, worin jede Formel B keinen homopolymeren Unterblock mit mehr als 7 Oxyalkylen-Einheiten hat.

12. Verfahren nach Anspruch 1, worin jede Formel B keinen homopolymeren Unterblock mit mehr als 4 Oxyalkylen-Einheiten hat.

13. Verfahren nach Anspruch 1, dadurch gekennzeichnet, daß die Einheiten der Formel I Teil eines Monomeren der Formel

$$L'\text{-}D\text{-}[A\text{-}L\text{-}D]_w\text{-}A\text{-}L'' \qquad (III)$$

sind, worin A und w wie in Anspruch 1 definiert sind, mit der Maßgabe, daß nicht alle A-Gruppen in irgendeinem Polymeren Homopolymere von Polyethylenglycol oder Polypropylenglycol sein können; worin jedes D unabhängig Sauerstoff oder -N($R^7$)- ist; jedes $R^7$ ist unabhängig ausgewählt unter Wasserstoff, $C_1$-$C_4$-Alkyl und Phenyl;
worin jedes L unabhängig ausgewählt ist unter -BRB′-; worin B, R und B′ wie in Anspruch 1 definiert sind; worin L′ ausgewählt ist unter Wasserstoff, P′-B-$R^x$-B′- und P′-$R^x$-B′-, worin jedes $R^x$ unabhängig ausgewählt ist unter

a) einer zweiwertigen aliphatischen Gruppe, vorzugsweise Alkyl, Alkenyl oder Alkinyl mit bis zu 25 Kohlenstoffatomen, die unterbrochen sein kann durch eine Unterbrechungseinheit, ausgewählt unter Oxy, Carbonyloxy, Amino, Aminocarbonyl, Oxycarbonyl, Ureido, Oxycarbonylamino und Carbonylamino;
b) einer zweiwertigen 5- bis 7-gliedrigen cycloaliphatischen Gruppe mit 5 bis 25 Kohlenstoffatomen;
c) einer zweiwertigen Arylengruppe mit 6 bis 25, vorzugsweise 7 bis 16 Kohlenstoffatomen;
d) einer zweiwertigen Aralkyl- oder Alkaryl-Gruppe mit 7 bis 25, vorzugsweise 8 bis 16 Kohlenstoffatomen;

worin die Gruppen b) und d) in ihren nichtcyclischen Teilen oder zwischen den cyclischen und nichtcyclischen Teilen unterbrochen sein können durch eine Unterbrechungseinheit, wie sie in a) definiert ist, und die Gruppen c) und d) können weiterhin substituiert sein in ihren Arylteilen mit einem oder mehreren Substituenten, ausgewählt unter Halogen, vorzugsweise Fluor oder Chlor, $C_1$-$C_4$-Alkyl, vorzugsweise Methyl, und $C_1$-$C_{12}$-Perhaloalkyl, insbesondere $C_1$-$C_{12}$-Perfluoralkyl;
mit der Carbonylgruppe von B sind sie gebunden an P′, und P′ ist Wasserstoff, Amino, Hydroxy oder ein Abschnitt, der eine vernetzbare Gruppe enthält, die vernetzt werden kann, wenn sie mit einem geeigneten Vernetzungsmittel coreagiert oder wenn sie durch aktinisches Licht bestrahlt wird;

und L″ ist ausgewählt unter Wasserstoff, L‴ wie nachfolgend definiert, $-B-R^x-B'-P'$ und $-B-R^x-P'$, worin $R^x$ und P′ wie oben definiert sind, mit Ausnahme dessen, daß das Carbonyl von B′ anstelle von B an P′ gebunden ist; und L‴ ist eine terminale einwertige aliphatische, aromatische oder cycloaliphatische Gruppe mit bis zu 14 Kohlenstoffatomen.

**14.** Verfahren nach Anspruch 13, worin P′ ein Vinyl-enthalten-der Teil ist.

**15.** Verfahren nach Anspruch 13, worin P′ die Bedeutung $H(R^b)C = C(R^a)$- hat, worin einer von $R^a$ und $R^b$ Wasserstoff und der andere Methyl oder Wasserstoff ist.

**16.** Ophthalmische Einrichtung, die ein vernetztes Polymer von Anspruch 1 umfaßt.

**17.** Ophthalmische Einrichtung nach Anspruch 16, dadurch gekennzeichnet, daß sie ein Polymer umfaßt wie in Anspruch 13 definiert.

**18.** Ophthalmische Einrichtung nach Anspruch 16, dadurch gekennzeichnet, daß sie eine Kontaktlinse ist.

**19.** Ophthalmische Einrichtung nach Anspruch 17, dadurch gekennzeichnet, daß sie eine Kontaktlinse ist.

**20.** Verfahren zur Korrektur eines Sehfehlers, gekennzeichnet durch die Anordnung der ophthalmischen Einrichtung von Anspruch 16 in die Blicklinie eines Auges, das einen solchen Fehler hat.

**21.** Verfahren nach Anspruch 20, gekennzeichnet durch Anordnung der ophthalmischen Einrichtung von Anspruch 17 in der Blicklinie eines Auges, das einen solchen Fehler hat.

**22.** Verwendung eines vernetzten Polymeren von Beispiel 1 zur Herstellung einer ophthalmischen Einrichtung.

**23.** Verwendung nach Anspruch 22, dadurch gekennzeichnet, daß die ophthalmische Einrichtung eine Kontaktlinse ist.

**Revendications**

**Revendications pour les Etats contractants suivants : AT, BE, CH, DE, FR, GB, GR, IT, LI, LU, NL, SE**

**1.** Polymère mouillable, souple, perméable à l'oxygène, réticulé, ayant des unités de formule

$$[-A-L-D-]_w \qquad (I)$$

dans laquelle chaque w est 1-8 ;
chaque D est -O- ou $-NR^7-$ dans lequel $R^7$ est un hydrogène, un alkyle inférieur, ou un phényle ;
chaque L est choisi indépendamment parmi -B-R-B′-, -BR- ou RB′-, dans lequels chaque B et B′ est choisi indépendamment parmi ces doubles liaisons :

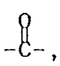, 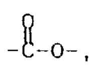, et 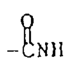

leur groupe carbonyle étant lié à A ou à D ;
chaque R est un groupe de liaison divalent choisi parmi

a) un groupe aliphatique divalent, pouvant avoir jusqu'à 25 atomes de carbone, qu'on peut interrompre par une unité d'interruption choisie parmi un oxy, carbonyloxy, amino, aminocarbonyle, oxycarbonyle, uréido, oxycarbonylamino, et carbonylamino ;
b) un groupe divalent $(aliphatique)_{alpha}$cycloaliphatique à 5-7 maillons-$(aliphatique)_{bêta}$ dans lequel chaque alpha et bêta représente indépendamment 0 ou 1, et chacun des groupes aliphatiques non-cycliques est choisi indépendamment dans le groupe a) ci-dessus, ledit groupe b) ayant jusqu'à 25 atomes de carbone ;
c) un groupe divalent $(aliphatique)_{alpha}$aryl-$(aliphatique)_{bêta}$ ayant de 6 à 25, de préférence 7 à 16 atomes de carbone dans les parties aryles, et chacun des groupes aliphatiques est choisi indépendamment

dans le groupe a) ci-dessus et alpha et bêta sont indépendamment 0 ou 1, dans lequel les groupes b) et c) peuvent être interrompus dans les parties non-cycliques ou entre la partie cyclique et la partie non-cyclique par un groupe d'interruption tel qu'il est défini en a) ci-dessus, et des groupes c) peuvent encore être substitués par un ou plusieurs substituants choisis parmi un halogène, un alkyle $C_{1-4}$, et un perhaloalkyle $C_{1-12}$ ;

et chaque A est une séquence copolymère de polyoxyalkylène, dont au moins 30%, des groupes A sont

aa) des blocs homopolymères de formule

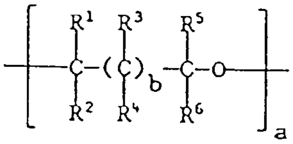

(A)

dans laquelle b représente 0 à 4, a peut avoir une valeur jusqu'à 15, et $R^1$ - $R^6$ sont tels que définis ci-dessous, et

ab) des séquences copolymères de formule

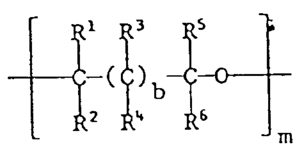

(B)

dans laquelle b est compris entre 0 et 4, $R^1$ - $R^6$ sont tels que définis ci-dessous,

et m peut atteindre 1000 à la condition que dans m, il n'y ait pas de séquence homopolymère de plus de 15 unités oxyalkylène, les groupes restants étant des homopolymères ou des copolymères de formule :

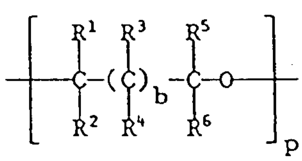

(C)

dans laquelle b est compris entre 0 et 4, p peut atteindre 1000 sans égard à la taille des sous-séquences homopolymères comprises dans p ; l'oxygène terminal dans le groupe A étant éventuellement remplacé par -$NR^7$-

et dans laquelle chacun des groupes $R^1$, $R^2$, $R^5$ et $R^6$ est choisi indépendamment dans le groupe constitué de l'hydrogène, d'un radical aliphatique, aromatique ou hétérocyclique choisi parmi un alkyle $C_{1-16}$ non-substitué ; un alkyle $C_{1-16}$ substitué, un alcényle $C_{2-16}$ non-substitué ; et un alcényle $C_{2-16}$ substitué ; dans lequel les substituants alkyles et alcényles sont choisis indépendamment parmi un alcoxycarbonyle $C_{1-16}$, un alcényloxycarbonyle $C_{2-16}$, un fluoro, un aryle pouvant avoir jusqu'à 10 atomes de carbone, un alcoxy $C_{1-8}$, un alcanoyloxy $C_{2-6}$, un aryloxy pouvant avoir jusqu'à 10 atomes de carbone, un alcénoyloxy $C_{3-6}$, aroyloxy pouvant avoir jusqu'à 11 atomes de carbone, un cycloalkyle $C_{3-8}$, un cycloalcoxy $C_{3-8}$, un cycloalkylcarbonyloxy $C_{3-8}$, un cycloalcoxycarbonyle $C_{3-8}$, un oxacycloalkyle pouvant avoir jusqu'à 7 atomes de carbone, un oxacycloalcoxy pouvant avoir jusqu'à 7 atomes de carbone, un oxacycloalcoxy (pouvant avoir jusqu'à 7 atomes de carbone)-carbonyle, un oxacycloalkyle (pouvant avoir jusqu'à 7 atomes de carbone)-carbonyloxy, et un aryle (pouvant avoir jusqu'à 10 atomes de carbone)-oxycarbonyle, chacun desdits substituants alkyle et alcényle étant, à son tour, éventuellement substitué par un alkyle $C_{1-6}$, un fluoro ou un alcoxy $C_{1-6}$, à la condition que ledit dernier alcoxy mentionné ne soit pas lié à un atome de carbone déjà fixé à un autre atome d'oxygène ; $R^1$, $R^2$, $R^5$ et $R^6$ étant choisis en outre indépendamment parmi un aryle jusqu'à 10 atomes de carbone, un cycloalkyle $C_{3-8}$, et un oxacycloalkyle pouvant avoir jusqu'à 7 atomes de carbone, chacun d'entre eux peut être non-substitué ou encore substitué par un substituant choisi dans le groupe des substituants dudit alkyle indiqué ci-dessus ;

$R^3$ et $R^4$ sont choisis dans le même groupe que celui indiqué ci-dessus pour $R^1$ ;

et $R^3$ et $R^4$ sont en outre indépendamment choisis parmi un alcoxycarbonyle $C_{1-16}$, un alcanoyloxy

$C_{2-16}$, un alcényloxycarbonyle $C_{2-16}$, et un alcénoyloxy $C_{3-16}$, dont chacun peut encore être substitué par un fluoro, aryle pouvant avoir jusqu'à 10 atomes de carbone, ou alcoxy $C_{1-16}$, et $R^3$ et $R^4$ sont encore indépendamment choisis parmi un aryloxy pouvant avoir jusqu'à 10 atomes de carbone, cycloalcoxy pouvant avoir jusqu'à 8 atomes de carbone, cycloalkyle (pouvant avoir jusqu'à 8 atomes de carbone)-carbonyloxy, un cycloalcoxy (pouvant avoir jusqu'à 8 atomes de carbone)-carbonyle, un aroyloxy pouvant avoir jusqu'à 11 atomes de carbone, un oxacycloalcoxy pouvant avoir jusqu'à 7 atomes de carbone, oxacycloalcényloxy pouvant avoir jusqu'à 7 atomes de carbone, un oxacycloalcoxy (pouvant avoir jusqu'à 7 atomes de carbone)-carbonyle, un oxacycloalkyle (pouvant avoir jusqu'à 7 atomes de carbone)-carbonyloxy, oxacycloalcényloxy (pouvant avoir jusqu'à 7 atomes de carbone)-carbonyle et aryloxy (pouvant avoir jusqu'à 10 atomes de carbone)-carbonyle, chacun d'entre eux peut être substitué en plus par un fluoro, alkyle $C_{1-6}$ ou alcoxy $C_{1-6}$, à la condition que l'un quelconque des substituants qui a un atome d'oxygène ou un groupe carbonyle comme liaison au reste de la molécule ne soit pas un substituant sur le même atome de carbone qui est fixé sur un autre atome d'oxygène, en plus, 2 groupes adjacents choisis parmi $R^1$ à $R^6$, avec les atomes auxquels ils sont fixés, peuvent former un cycloalkyle à 5-8 maillons, un oxacycloalkyle à 5-8 maillons, ou un cycle bicycloalkyle, de telle sorte que le polymère résultant absorbe plus de 10% en poids d'eau par rapport au poids total de polymère.

**2.** Polymère de la revendication 1 dans lequel au moins 50% desdits groupes -A- sont choisis parmi la formule A et la formule B.

**3.** Polymère de la revendication 1, dans lequel au moins 75% desdits groupes -A- sont choisis parmi la formule A et la formule B.

**4.** Polymère de la revendication 1, dans lequel tous les groupes -A- sont choisis parmi la formule A et la formule B.

**5.** Polymère de la revendication 1, dans lequel lesdits groupes -A- sont choisis parmi la formule A et la formule C.

**6.** Polymère de la revendication 1, dans lequel lesdits groupes -A- sont choisis parmi la formule B et la formule C.

**7.** Polymère de la revendication 1, dans lequel chaque a n'est pas supérieur à 10.

**8.** Polymère de la revendication 1, dans lequel chaque a n'est pas supérieur à 7.

**9.** Polymère de la revendication 1, dans lequel chaque a n'est pas supérieur à 4.

**10.** Polymère selon la revendication 1, dans lequel chaque formule B n'a pas de sous-séquence homopolymère ayant plus de 10 unités oxyalkylène.

**11.** Polymère selon la revendication 1, dans lequel chaque formule B n'a pas de sous-séquence homopolymère ayant plus de 7 unités oxyalkylène.

**12.** Polymère selon la revendication 1, dans lequel chaque formule B n'a pas de sous-séquence homopolymère ayant plus de 4 unités oxyalkylène.

**13.** Polymère de la revendication 1, caractérisé en ce que les unités de formule I font partie du monomère de formule

$$L'\text{-}D\text{-}[A\text{-}L\text{-}D]_w\text{-}A\text{-}L'' \qquad \text{(III)}$$

dans laquelle A et w sont tels que définis à la revendication 1, à la condition que tous les groupes A dans l'un quelconque des polymères ne peuvent pas être homopolymères de polyéthylène glycol ou de polypropylène glycol ;

dans laquelle chaque D représente indépendamment l'oxygène ou $-N(R^7)-$ ; chaque $R^7$ est choisi indépendamment parmi un hydrogène, un alkyle $C_{1-4}$ et un phényle ;

dans laquelle chaque L est choisi indépendamment parmi -BRB'- ; dans laquelle B, R et B' sont tels que définis ci-dessus ;

dans laquelle L' est choisi parmi un hydrogène, $P'\text{-}B\text{-}R^x\text{-}B'\text{-}$ et $P'\text{-}R^x\text{-}B'\text{-}$, dans laquelle chaque $R^x$ est choisi indépendamment de

a) un groupe aliphatique divalent, de préférence alkyle, alcényle ou alcynyle, pouvant avoir jusqu'à 25

atomes de carbone, qui peut être interrompu par une unité d'interruption choisie parmi un groupe oxy, carbonyloxy, amino, aminocarbonyle, oxycarbonyle, uréido, oxycarbonylamino, et carbonylamino ;
b) un groupe cycloaliphatique à 5-7 maillons divalent ayant 5 à 25 atomes de carbone ;
c) un groupe arylène divalent ayant 6 à 25, de préférence 7-16 atomes de carbone ;
d) un groupe aralkyle ou alcaryle divalent ayant de 7 à 25, de préférence 8 à 16 atomes de carbone ;
où les groupes b) et d) peuvent être interrompus dans les parties non-cycliques ou entre la partie cyclique et la partie non-cyclique par un groupe d'interruption tel qu'il est défini en a), ci-dessus, et des groupes c) et d) peuvent encore être substitués dans les portions aryles par un ou plusieurs substituants choisis parmi les halogènes, de préférence le fluor ou le chlore, un alkyle $C_{1-4}$, de préférence un méthyle, et un perhaloalkyle $C_{1-12}$, spécialement un perfluoro alkyle $C_{1-12}$ ;

avec le groupe carbonyle de B qui est lié à P′ et P′ est un hydrogène, amino, hydroxy ou un radical contenant un groupe réticulable qu'on peut réticuler quand on le fait réagir avec un agent de réticulation approprié ou quand on l'irradie par une radiation actinique ;

et L″ est choisi parmi un hydrogène, L‴ est tel que défini ci-dessous, -B-$R^x$-B′-P′ et -B-$R^x$-P′, dans lesquels $R^x$ et P′ sont tels que définis ci-dessus, sauf que le radical carbonyle de B′ au lieu de B est lié à P′, et L‴ est un groupe terminal monovalent aliphatique aromatique ou cycloaliphatique pouvant avoir jusqu'à 14 atomes de carbone.

14. Polymère de la revendication 13, dans lequel P′ est un radical contenant un vinyle.

15. Polymère selon la revendication 13, dans lequel P′ est $H(R^b)C=C(R^a)$- dans laquelle l'un des groupes $R^a$ et $R^b$ est un hydrogène et l'autre est un méthyle ou un hydrogène.

16. Dispositif ophtalmique qui comprend un polymère réticulé selon la revendication 1.

17. Dispositif ophtalmique selon la revendication 16, caractérisé en ce qu'il comprend un polymère tel qu'il est défini à la revendication 13.

18. Dispositif ophtalmique selon la revendication 16, caractérisé en ce que c'est une lentille de contact.

19. Dispositif ophtalmique selon la revendication 17, caractérisé en ce que c'est une lentille de contact.

20. Procédé de correction d'un défaut visuel qui comprend de placer un dispositif ophtalmique selon la revendication 16 dans la ligne de vue d'un oeil qui a un tel défaut.

21. Procédé selon la revendication 20, caractérisé en ce qu'on place un dispositif ophtalmique selon la revendication 17 dans la ligne de vue d'un oeil qui a un tel défaut.

22. Utilisation d'un polymère réticulé selon la revendication 1 pour fabriquer un dispositif ophtalmique.

23. Utilisation selon la revendication 22, caractérisée en ce que le dispositif ophtalmique est une lentille de contact.

**Revendications pour l'Etat contractant suivant : ES**

1. Procédé de fabrication d'un polymère mouillable, souple, perméable à l'oxygène, réticulé, ayant des unités de formule

$$[A\text{-}L\text{-}D]_w \qquad (I)$$

dans laquelle chaque w est 1-8 ;
chaque D est -O- ou -$NR^7$- dans lequel $R^7$ est un hydrogène, un alkyle inférieur, ou un phényle ;
chaque L est choisi indépendamment parmi -B-R-B′-, -BR- ou RB′-, dans lequels chaque B et B′ est choisi indépendamment parmi ces doubles liaisons :

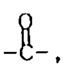, 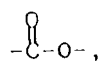, et 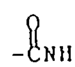

leur groupe carbonyle étant lié à A ou à D ;
chaque R est un groupe de liaison divalent choisi parmi

a) un groupe aliphatique divalent, pouvant avoir jusqu'à 25 atomes de carbone, qu'on peut interrompre par une unité d'interruption choisie parmi un oxy, carbonyloxy, amino, aminocarbonyle, oxycarbonyle, uréido, oxycarbonylamino, et carbonylamino ;

b) un groupe divalent $(aliphatique)_{alpha}$cycloaliphatique à 5-7 maillons-$(aliphatique)_{bêta}$ dans lequel chaque alpha et bêta représente indépendamment O ou 1, et chacun des groupes aliphatiques non-cycliques est choisi indépendamment dans le groupe a) ci-dessus, ledit groupe b) ayant jusqu'à 25 atomes de carbone ;

c) un groupe divalent $(aliphatique)_{alpha}$aryl-$(aliphatique)_{bêta}$ ayant de 6 à 25, de préférence 7 à 16 atomes de carbone dans les parties aryles, et chacun des groupes aliphatiques est choisi indépendamment dans le groupe a) ci-dessus et alpha et bêta sont indépendamment 0 ou 1, dans lequel les groupes b) et c) peuvent être interrompus dans les parties non-cycliques ou entre la partie cyclique et la partie non-cyclique par un groupe d'interruption tel qu'il est défini en a) ci-dessus, et des groupes c) peuvent encore être substitués par un ou plusieurs substituants choisis parmi un halogène, un alkyle $C_{1-4}$, et un perhaloalkyle $C_{1-12}$ ;

et chaque A est une séquence copolymère de polyoxyalkylène, dont au moins 30%, des groupes A sont

aa) des blocs homopolymères de formule

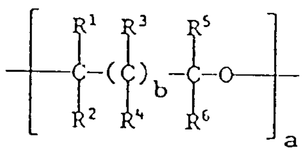

(A)

dans laquelle b représente 0 à 4, a peut avoir une valeur jusqu'à 15, et $R^1$ - $R^6$ sont tels que définis ci-dessous, et

ab) des séquences copolymères de formule

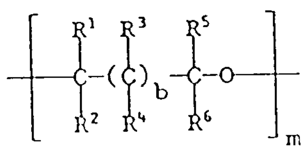

(B)

dans laquelle b est compris entre 0 et 4, $R^1$ - $R^6$ sont tels que définis ci-dessous,

et m peut atteindre 1000 à la condition que dans m, il n'y ait pas de séquence homopolymère de plus de 15 unités oxyalkylène, les groupes restants étant des homopolymères ou des copolymères de formule :

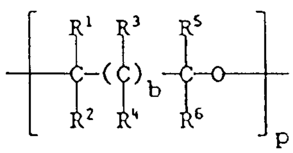

(C)

dans laquelle b est compris entre 0 et 4, p peut atteindre 1000 sans égard à la taille des sous-séquences homopolymères comprises dans p ; l'oxygène terminal dans le groupe A étant éventuellement remplacé par $-NR^7-$ et dans laquelle chacun des groupes $R^1$, $R^2$, $R^5$ et $R^6$ est choisi indépendamment dans le groupe constitué de l'hydrogène, d'un radical aliphatique, aromatique ou hétérocyclique choisi parmi un alkyle $C_{1-16}$ non-substitué ; un alkyle $C_{1-16}$ substitué, un alcényle $C_{2-16}$ non-substitué ; et un alcényle $C_{2-16}$ substitué ; dans lequel les substituants alkyles et alcényles sont choisis indépendamment parmi un alcoxycarbonyle $C_{1-16}$, un alcényloxycarbonyle $C_{2-16}$, un fluoro, un aryle pouvant avoir jusqu'à 10 atomes de carbone, un alcoxy $C_{1-8}$, un alcanoyloxy $C_{2-6}$, un aryloxy pouvant avoir jusqu'à 10 atomes de carbone, un alcénoyloxy $C_{3-6}$, aroyloxy pouvant avoir jusqu'à 11 atomes de carbone, un cycloalkyle $C_{3-8}$, un cycloalcoxy $C_{3-8}$, un cycloalkylcarbonyloxy $C_{3-8}$, un cycloalcoxycarbonyle $C_{3-8}$, un oxacycloalkyle pouvant avoir jusqu'à 7 atomes de carbone, un oxacycloalcoxy pouvant avoir jusqu'à 7 atomes de carbone, un oxacycloalcoxy (pouvant avoir jusqu'à 7 atomes de carbone)-carbonyle, un oxacycloalkyle (pouvant avoir jusqu'à 7 atomes de carbone)-carbonyloxy, et un aryle (pouvant avoir jusqu'à 10 atomes de carbone)-oxycarbonyle, chacun desdits

substituants alkyle et alcényle étant, à son tour, éventuellement substitué par un alkyle $C_{1-6}$, un fluoro ou un alcoxy $C_{1-6}$, à la condition que ledit dernier alcoxy mentionné ne soit pas lié à un atome de carbone déjà fixé à un autre atome d'oxygène ; $R^1$, $R^2$, $R^5$ et $R^6$ étant choisis en outre indépendamment parmi un aryle jusqu'à 10 atomes de carbone, un cycloalkyle $C_{3-8}$, et un oxacycloalkyle pouvant avoir jusqu'à 7 atomes de carbone, chacun d'entre eux peut être non-substitué ou encore substitué par un substituant choisi dans le groupe des substituants dudit alkyle indiqué ci-dessus ;

$R^3$ et $R^4$ sont choisis dans le même groupe que celui indiqué ci-dessus pour $R^1$ ;

et $R^3$ et $R^4$ sont en outre indépendamment choisis parmi un alcoxycarbonyle $C_{1-16}$, un alcanoyloxy $C_{2-16}$, un alcényloxycarbonyle $C_{2-16}$, et un alcénoyloxy $C_{3-16}$, dont chacun peut encore être substitué par un fluoro, aryle pouvant avoir jusqu'à 10 atomes de carbone, ou alcoxy $C_{1-16}$, et $R^3$ et $R^4$ sont encore indépendamment choisis parmi un aryloxy pouvant avoir jusqu'à 10 atomes de carbone, cycloalcoxy pouvant avoir jusqu'à 8 atomes de carbone, cycloalkyle (pouvant avoir jusqu'à 8 atomes de carbone)-carbonyloxy, un cycloalcoxy (pouvant avoir jusqu'à 8 atomes de carbone)-carbonyle, un aroyloxy pouvant avoir jusqu'à 11 atomes de carbone, un oxacycloalcoxy pouvant avoir jusqu'à 7 atomes de carbone, oxacycloalcényloxy pouvant avoir jusqu'à 7 atomes de carbone, un oxacycloalcoxy (pouvant avoir jusqu'à 7 atomes de carbone)-carbonyle, un oxacycloalkyle (pouvant avoir jusqu'à 7 atomes de carbone)-carbonyloxy, oxacycloalcényloxy (pouvant avoir jusqu'à 7 atomes de carbone)-carbonyle et aryloxy (pouvant avoir jusqu'à 10 atomes de carbone)-carbonyle, chacun d'entre eux peut être substitué en plus par un fluoro, alkyle $C_{1-6}$ ou alcoxy $C_{1-6}$, à la condition que l'un quelconque des substituants qui a un atome d'oxygène ou un groupe carbonyle comme liaison au reste de la molécule ne soit pas un substituant sur le même atome de carbone qui est fixé sur un autre atome d'oxygène, en plus, 2 groupes adjacents choisis parmi $R^1$ à $R^6$, avec les atomes auxquels ils sont fixés, peuvent former un cycloalkyle à 5-8 maillons, un oxacycloalkyle à 5-8 maillons, ou un cycle bicycloalkyle, de telle sorte que le polymère résultant absorbe plus de 10% en poids d'eau par rapport au poids total de polymère, en réticulant d'une manière déjà connue.

**2.** Procédé de la revendication 1 dans lequel au moins 50% desdits groupes -A- sont choisis parmi la formule A et la formule B.

**3.** Procédé de la revendication 1, dans lequel au moins 75% desdits groupes -A- sont choisis parmi la formule A et la formule B.

**4.** Procédé de la revendication 1, dans lequel tous les groupes -A- sont choisis parmi la formule A et la formule B.

**5.** Procédé de la revendication 1, dans lequel lesdits groupes -A- sont choisis parmi la formule A et la formule C.

**6.** Procédé de la revendication 1, dans lequel lesdits groupes -A- sont choisis parmi la formule B et la formule C.

**7.** Procédé de la revendication 1, dans lequel chaque a n'est pas supérieur à 10.

**8.** Procédé de la revendication 1, dans lequel chaque a n'est pas supérieur à 7.

**9.** Procédé de la revendication 1, dans lequel chaque a n'est pas supérieur à 4.

**10.** Procédé selon la revendication 1, dans lequel chaque formule B n'a pas de sous-séquence homopolymère ayant plus de 10 unités oxyalkylène.

**11.** Procédé selon la revendication 1, dans lequel chaque formule B n'a pas de sous-séquence homopolymère ayant plus de 7 unités oxyalkylène.

**12.** Procédé selon la revendication 1, dans lequel chaque formule B n'a pas de sous-séquence homopolymère ayant plus de 4 unités oxyalkylène.

**13.** Procédé de la revendication 1, caractérisé en ce que les unités de formule I font partie du monomère de formule

$$L'\text{-}D\text{-}[A\text{-}L\text{-}D]_w\text{-}A\text{-}L'' \qquad (III)$$

dans laquelle A et w sont tels que définis à la revendication 1, à la condition que tous les groupes

A dans l'un quelconque des polymères ne peuvent pas être homopolymères de polyéthylène glycol ou de polypropylène glycol ;

dans laquelle chaque D représente indépendamment l'oxygène ou -N($R^7$)- ; chaque $R^7$ est choisi indépendamment parmi un hydrogène, un alkyle $C_{1-4}$ et un phényle ;

dans laquelle chaque L est choisi indépendamment parmi -BRB'- ; dans laquelle B, R et B' sont tels que définis ci-dessus ;

dans laquelle L' est choisi parmi un hydrogène, P'-B-$R^x$-B'- et P'-$R^x$-B'-, dans laquelle chaque $R^x$ est choisi indépendamment de

a) un groupe aliphatique divalent, de préférence alkyle, alcényle ou alcynyle, pouvant avoir jusqu'à 25 atomes de carbone, qui peut être interrompu par une unité d'interruption choisie parmi un groupe oxy, carbonyloxy, amino, aminocarbonyle, oxycarbonyle, uréido, oxycarbonylamino, et carbonylamino ;
b) un groupe cycloaliphatique à 5-7 maillons divalent ayant 5 à 25 atomes de carbone ;
c) un groupe arylène divalent ayant 6 à 25, de préférence 7-16 atomes de carbone ;
d) un groupe aralkyle ou alcaryle divalent ayant de 7 à 25, de préférence 8 à 16 atomes de carbone ;
où les groupes b) et d) peuvent être interrompus dans les parties non-cycliques ou entre la partie cyclique et la partie non-cyclique par un groupe d'interruption tel qu'il est défini en a), ci-dessus, et des groupes c) et d) peuvent encore être substitués dans les portions aryles par un ou plusieurs substituants choisis parmi les halogènes, de préférence le fluor ou le chlore, un alkyle $C_{1-4}$, de préférence un méthyle, et un perhaloalkyle $C_{1-12}$, spécialement un perfluoro alkyle $C_{1-12}$ ;

avec le groupe carbonyle de B qui est lié à P' et P' est un hydrogène, amino, hydroxy ou un radical contenant un groupe réticulable qu'on peut réticuler quand on le fait réagir avec un agent de réticulation approprié ou quand on l'irradie par une radiation actinique ;

et L" est choisi parmi un hydrogène, L''' est tel que défini ci-dessous, -B-$R^x$-B'-P' et -B-$R^x$-P', dans lesquels $R^x$ et P' sont tels que définis ci-dessus, sauf que le radical carbonyle de B' au lieu de B est lié à P', et L''' est un groupe terminal monovalent aliphatique aromatique ou cycloaliphatique pouvant avoir jusqu'à 14 atomes de carbone.

**14.** Procédé de la revendication 13, dans lequel P' est un radical contenant un vinyle.

**15.** Procédé selon la revendication 13, dans lequel P' est H($R^b$)C=C($R^a$)- dans laquelle l'un des groupes $R^a$ et $R^b$ est un hydrogène et l'autre est un méthyle ou un hydrogène.

**16.** Dispositif ophtalmique qui comprend un polymère réticulé selon la revendication 1.

**17.** Dispositif ophtalmique selon la revendication 16, caractérisé en ce qu'il comprend un polymère tel qu'il est défini à la revendication 13.

**18.** Dispositif ophtalmique selon la revendication 16, caractérisé en ce que c'est une lentille de contact.

**19.** Dispositif ophtalmique selon la revendication 17, caractérisé en ce que c'est une lentille de contact.

**20.** Procédé de correction d'un défaut visuel qui comprend de placer un dispositif ophtalmique selon la revendication 16 dans la ligne de vue d'un oeil qui a un tel défaut.

**21.** Procédé selon la revendication 20, caractérisé en ce qu'on place un dispositif ophtalmique selon la revendication 17 dans la ligne de vue d'un oeil qui a un tel défaut.

**22.** Utilisation d'un polymère réticulé selon la revendication 1 pour fabriquer un dispositif ophtalmique.

**23.** Utilisation selon la revendication 22, caractérisée en ce que le dispositif ophtalmique est une lentille de contact.